# EUROPEAN PATENT APPLICATION

(11) **EP 4 537 780 A1**
(43) Date of publication of application: **16.04.2025**
(21) Application number: 22947571.0
(22) Date of filing: 09.08.2022
(51) Int. Cl.: A61B 34/10, G06T 7/00, G06T 7/11, G06T 17/00

(54) **PATH PLANNING APPARATUS AND METHOD, SURGICAL SYSTEM, AND COMPUTER-READABLE STORAGE MEDIUM**

(30) Priority: 22.06.2022 CN 202210715863
(71) Applicant: SceneRay Co., Ltd., Jiangsu 215123 (CN)
(72) Inventor: CHEN, Lei, Suzhou, Jiangsu 215123 (CN); ZHU, Weiran, Suzhou, Jiangsu 215123 (CN); NING, Yihua, Suzhou, Jiangsu 215123 (CN)
(74) Representative: Cabinet Beaumont
(86) International application number: PCT/CN2022/111224
(87) International publication number: WO 2023/245830

(57) **Abstract**

Provided are a path planning apparatus (10, 200) and method, a surgical system and a computer-readable storage medium. The path planning apparatus (10, 200) includes a processor (220) configured to plan a path through which an electrode lead is implanted into an intracranial region of a patient, where the planned path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient (S101). The electrode lead is configured to deliver electrical stimulation to the anterior limb of the internal capsule and the nucleus accumbens. The path planned by the path planning apparatus (10, 200) enables the electrode lead to be in contact with an anterior limb of an internal capsule and a nucleus accumbens of one hemisphere. In this manner, the electrode lead can simultaneously deliver electrical stimulation to the anterior limb of the internal capsule and the nucleus accumbens, and a joint electrical stimulation therapy is achieved, having a relatively good therapeutical effect on diseases such as addictive behavior, depressive disorder and obsessive-compulsive disorder.

## Description

This application claims priority to Chinese Patent Application No. 202210715863.1 filed Jun. 22, 2022, the disclosure of which is incorporated herein by reference in its entirety.

### TECHNICAL FIELD

The present application relates to the technical field of implantable devices and, in particular, to a path planning apparatus and method, a surgical system and a computer-readable storage medium.

### BACKGROUND

With the development of science and technology and social progress, a patient is eager to improve quality of life through various treatment means, among which implantable devices have a very broad application prospect. Implantable devices refer to medical devices that enter human bodies or cavities (mouths) in whole or in part by means of surgeries, or are used for replacing human epithelial surfaces or eye surfaces, and are left in the human bodies for 30 days (inclusive) or more or absorbed by the human bodies after surgery processes end. Stimulators are a type of the implantable devices. A stimulator, which generally includes an IPG, an extended lead and an electrode lead, can provide a patient with a refined electrical stimulation therapy with controllable parameters and is popular with many consumers in the market.

In recent years, deep brain stimulation (DBS) technology has been tried out for relapse prevention treatment of drug addiction. However, the intervention of DBS on a specific target site in a brain, the adaptability of a DBS apparatus to the target site and a mode of a stimulus output of the DBS are still not clear or are blank fields.

The patent CN111588463A discloses a method and apparatus for determining a surgical plan and a storage medium. The method includes: acquiring an object tissue image of a target object; acquiring a white matter tract image of the target object; performing image fusion on the object tissue image and the white matter tract image to obtain a fused object image; and displaying the fused object image. The fused object image includes a content of the object tissue image and a content of the white matter tract image to plan a surgical path in conjunction with the content of the object tissue image and the content of the white matter tract image. The method does not limit a specific path obtained after the planning.

Based on this, the present application provides a path planning apparatus and method, a surgical system and a computer-readable storage medium to solve the problems in the above existing art.

### SUMMARY

An object of the present application is to provide a path planning apparatus and method, a surgical system and a computer-readable storage medium for planning an implantation path of an electrode lead so that the electrode lead performs joint electrical stimulation on a nucleus accumbens and an anterior limb of an internal capsule.

The object of the present application is achieved through the technical solutions described below.

In a first aspect, the present application provides a path planning apparatus. The path planning apparatus includes a processor configured to perform the operation described below.

A path through which an electrode lead is implanted into an intracranial region of a patient is planned, where the planned path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient.

The electrode lead is configured to deliver electrical stimulation to the anterior limb of the internal capsule and the nucleus accumbens.

An implantation path of a particular electrode lead is planned, and the electrode lead is implanted into one hemisphere of the brain of the patient (for example, a left hemisphere or a right hemisphere) according to the path so that joint electrical stimulation can be performed on a nucleus accumbens and an anterior limb of an internal capsule of this hemisphere to treat the patient. The path planned by the path planning apparatus enables the electrode lead to be in contact with an anterior limb of an internal capsule and a nucleus accumbens of one hemisphere. In this manner, the electrode lead can simultaneously deliver electrical stimulation to the anterior limb of the internal capsule and the nucleus accumbens, and a joint electrical stimulation therapy is achieved. In a DBS field, doctors performed an electrical stimulation therapy on only one particular brain tissue in the past. Therefore, after one electrode lead was implanted into an intracranial region of a patient, even though multiple electrode patches may be on the electrode lead, these electrode patches were used for delivering electrical stimulation to the same brain tissue (nucleus) and cannot simultaneously deliver electrical stimulation to multiple brain tissues, and no joint electrical stimulation therapy was performed on two particular brain tissues (an anterior limb of an internal capsule and a nucleus accumbens). The path planning apparatus provided in the present application can plan the implantation path of the electrode lead so that after the electrode lead is implanted into the intracranial region of the patient, a joint electrical stimulation therapy can be performed on an anterior limb of an internal capsule and a nucleus accumbens of one hemisphere of the brain, having a relatively good therapeutical effect on diseases such as addictive behavior, depressive disorder and obsessive-compulsive disorder.

In some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

The electrode lead passes through the anterior limb of the internal capsule and the nucleus accumbens in a particular direction, and the joint electrical stimulation for the anterior limb of the internal capsule and the nucleus accumbens is used for the treatment of a particular type of disease, for example, the treatment of multiple mental disorders, thereby effectively alleviating a condition of the patient or even curing a disease of the patient.

In some possible implementations, the processor is configured to plan the path in the manners described below.

Image data of the brain of the patient is acquired, where the image data includes magnetic resonance image data and/or CT image data.

An extreme point of a ventral contour of the nucleus accumbens is positioned according to the image data to obtain positioning data of the extreme point.

The path through which the electrode lead is implanted into the intracranial region of the patient is planned according to the positioning data of the extreme point so that a straight line where the path is located passes through the extreme point from a superior side to an inferior side, from an anterior side to a posterior side and from a lateral side to a medial side.

The image data is used for positioning the extreme point of the ventral contour of the nucleus accumbens, and the extreme point is used for planning the implantation path of the electrode lead so that the straight line where the planned path is located passes through the extreme point.

In some possible implementations, the processor is configured to position the straight line in the manners described below.

A set point of the anterior limb of the internal capsule is positioned according to the image data to obtain positioning data of the set point.

The straight line is positioned according to the positioning data of the set point and the positioning data of the extreme point so that the straight line passes through the set point and the extreme point.

The set point of the anterior limb of the internal capsule and the extreme point of the nucleus accumbens are used for forming a constraint on the path, and the implantation path of the electrode lead is planned in a two-point-one-line manner so that the electrode lead sequentially passes through the anterior limb of the internal capsule and the nucleus accumbens in an implantation process from a superior side to an inferior side, from an anterior side to a posterior side and from a lateral side to a medial side.

In some possible implementations, the processor is configured to position a set point of the anterior limb of the internal capsule in the manners described below.

A segmentation result of the anterior limb of the internal capsule is acquired according to the image data, where the segmentation result of the anterior limb of the internal capsule is configured to indicate multiple regions of the anterior limb of the internal capsule.

One of the multiple regions is determined as a target region according to a projection relationship between each of the multiple regions and a frontal lobe so that a white matter tract traveling in the target region can be projected into a target prefrontal cortex, where the target frontal cortex is a frontal cortex associated with the disease of the patient.

The set point of the anterior limb of the internal capsule is positioned from the target region.

The image data is used for acquiring the segmentation result of the anterior limb of the internal capsule, and the segmentation result of the anterior limb of the internal capsule is used for positioning the set point of the anterior limb of the internal capsule. When the implantation path passes through the set point, the electrode lead can release electrical stimulation energy to a contact portion (the target region) of the anterior limb of the internal capsule with the electrode lead. This energy is projected into the target prefrontal cortex via the white matter tract in the target region and accurately acts on a particular prefrontal cortex associated with the disease of the patient, thereby achieving the precise treatment of a mental disorder.

In some possible implementations, the processor is configured to position the straight line in the manners described below.

The straight line is enabled to be in a reference plane, and an acute included angle formed between the straight line and any transverse plane of the brain is enabled to be a second preset angle.

The reference plane satisfies the following conditions: the extreme point is in the reference plane, an acute included angle formed between the reference plane and any sagittal plane of the brain is a first preset angle, and a normal of the reference plane is parallel to any coronal plane of the brain.

The straight line where the path is located is located in a particular reference plane, and a particular acute included angle (the second preset angle) is formed between the straight line and any transverse plane of the brain. The setting of the reference plane needs to satisfy multiple conditions. Not only the extreme point is set in the reference plane, but also a particular acute included angle (the first preset angle) is formed between the reference plane and any sagittal plane, and the normal of the reference plane is parallel to the coronal plane, that is, a projection of the reference plane on the coronal plane is a straight line. In this manner, the straight line where the planned path is located can pass through the anterior limb of the internal capsule and the nucleus accumbens in a particular direction and at a particular angle. Therefore, the electrode lead implanted according to such a path can pass through the anterior limb of the internal capsule and the nucleus accumbens in the particular direction and at the particular angle, thereby performing the joint electrical stimulation therapy on the two brain tissues.

In some possible implementations, the first preset angle is not less than 5 degrees and is not greater than 85 degrees, and the second preset angle is not less than 15 degrees and is less than 90 degrees.

Since a brain structure varies with each individual and a brain structure of each person is different, the first preset angle and the second preset angle may be values within a relatively wide value range, provided that joint electrical stimulation can be performed on an anterior limb of an internal capsule and a nucleus accumbens of one hemisphere of the brain of the patient.

In some possible implementations, the first preset angle is not less than 15 degrees and is not greater than 45 degrees, and the second preset angle is not less than 40 degrees and is not greater than 85 degrees.

Although a brain structure of each person is different, brain structures of most people are relatively similar. In terms of probability, a more accurate angle range can be preferentially used to quickly determine a direction of the implantation path, which is sufficient to cope with a general situation of most people. For a very small number of people whose brain structures are very different from those of ordinary people, a wider range can be enabled as an alternative to help doctors plan a path for these few people to implant electrode leads.

In some possible implementations, the processor is configured to determine the first preset angle and the second preset angle in the manners described below.

A set point of the anterior limb of the internal capsule is positioned according to the image data to obtain positioning data of the set point.

The first preset angle and the second preset angle are determined according to the positioning data of the set point and the positioning data of the extreme point.

Before the first preset angle, the second preset angle and the extreme point are used for determining the implantation path, the set point of the anterior limb of the internal capsule and the extreme point of the nucleus accumbens are used as the constraint, the value range of the first preset angle and the second preset angle are significantly narrowed, and a range where the implantation path of the electrode lead is located in the implantation process is significantly narrowed. In particular, when the first preset angle and the second preset angle may be values within a relatively wide value range, an amount of data for calculating all feasible solutions of the first preset angle and the second preset angle can be significantly reduced.

In some possible implementations, the processor is configured to position the extreme point of the ventral contour of the nucleus accumbens in the manners described below.

A reference point is positioned according to the image data, and a two-dimensional image of the brain corresponding to the reference point is generated, where the two-dimensional image of the brain corresponding to the reference point includes one or more of a two-dimensional image of a coronal plane, a two-dimensional image of a sagittal plane and a two-dimensional image of a transverse plane.

The extreme point of the ventral contour of the nucleus accumbens is positioned by using the two-dimensional image of the brain corresponding to the reference point.

The image data is used for positioning the reference point. The reference point may be, for example, an anterior commissure, a posterior commissure or a midpoint of the anterior commissure and the posterior commissure. After the reference point is positioned, a coronal plane, a sagittal plane and a transverse plane of the brain corresponding to the reference point can be positioned, that is, a coronal plane passing through the reference point (in other words, the reference point is in the coronal plane), a sagittal plane passing through the reference point and a transverse plane passing through the reference point, and three-dimensional image data is used for generating one or more of the two-dimensional images corresponding to the coronal plane, the sagittal plane and the transverse plane. Since the ventral contour of the nucleus accumbens presents a parabola-like shape in the above two-dimensional image (an opening of the parabola is upward and outward), the extreme point of the ventral contour of the nucleus accumbens can be positioned from the parabola. Moreover, the step of positioning the extreme point may be achieved in an intelligent recognition or manual calibration manner. For doctors, the manner for positioning the extreme point is very intuitive, and the extreme point can be quickly calibrated according to experience. In the intelligent recognition manner, the contour of the nucleus accumbens can be quickly recognized through an edge detection algorithm and/or an image segmentation algorithm, thereby positioning the extreme point of the ventral contour of the nucleus accumbens. The path planning apparatus provided in the present application generates the two-dimensional image by using the reference point and positions the extreme point by using the two-dimensional image. This manner of positioning the extreme point has a simple calculation process, a small calculation amount and a fast calculation speed and consumes less calculation resources.

In some possible implementations, the reference point is a midpoint of a connecting line between an anterior commissure and a posterior commissure of the brain.

Alternatively, the reference point is an anterior commissure of the brain.

Alternatively, the reference point is a posterior commissure of the brain.

Using the anterior commissure of the brain, the posterior commissure of the brain or the midpoint of the connecting line between the anterior commissure and the posterior commissure of the brain as the reference point has the advantages of simple operation and easy implementation in the intelligent recognition manner or the manual calibration manner. For doctors, the manner for positioning the reference point is very intuitive, and the reference point can be quickly calibrated according to experience. In the intelligent recognition manner, a contour of an entire brain can be quickly obtained through the edge detection algorithm, and the anterior commissure and the posterior commissure are positioned from the contour of the entire brain, thereby positioning the reference point.

In some possible implementations, the processor is configured to position the extreme point of the ventral contour of the nucleus accumbens in the manners described below.

An adjustment operation for each two-dimensional image is received, and in response to the adjustment operation, one or more of the following parameters of the each two-dimensional image are adjusted: brightness, contrast, saturation, exposure, hue, grayscale and scaling.

The extreme point of the ventral contour of the nucleus accumbens is positioned by using the adjusted two-dimensional image.

Doctors can manually adjust the parameter of the two-dimensional image. Adjusting the parameter can change a display effect of the two-dimensional image, thereby facilitating determining the extreme point from the two-dimensional image. The adjusted parameter is not limited in the present application and may be, for example, one or more of multiple parameters that affect the display effect. Apparently, the adjustment to one or more of these parameters can make the ventral contour of the nucleus accumbens more convex and clearer (in other words, the contrast is higher) so that the positioned extreme point is more accurate and reliable, the positioning speed is faster and the positioning efficiency is high, thereby improving overall path planning efficiency.

In some possible implementations, the adjustment operation is an operation of adjusting a parametric curve and is configured to adjust the brightness, the contrast or the grayscale of the each two-dimensional image.

Alternatively, the adjustment operation is an operation of adjusting a parameter progress bar and is configured to adjust the brightness, the contrast, the saturation, the exposure, the hue, the grayscale and the scaling of the each two-dimensional image.

Alternatively, the adjustment operation is an operation of adjusting a parameter value and is configured to adjust the brightness, the contrast, the saturation, the exposure, the hue, the grayscale and the scaling of the each two-dimensional image.

Doctors are allowed to adjust the brightness or the contrast in a manner of adjusting the parametric curve, the doctors are allowed to adjust multiple parameters in a manner of adjusting the parameter progress bar, or the doctors are allowed to adjust the multiple parameters in a manner of adjusting the parameter value so that the doctors can select a manner of adjusting the parameters according to preferences of the doctors to quickly adjust the display effect of the two-dimensional image, thereby further improving the path planning efficiency.

In some possible implementations, the processor is configured to position the extreme point of the ventral contour of the nucleus accumbens in the manners described below.

A three-dimensional model of the brain of the patient is generated according to the image data.

The extreme point of the ventral contour of the nucleus accumbens is positioned by using the three-dimensional model.

The image data is used for generating the three-dimensional model of the brain, and the three-dimensional model is used for positioning the extreme point. The manner of positioning the extreme point may be intelligent recognition or manual calibration. When the manual calibration is used, compared with the image data and the two-dimensional image, the three-dimensional model has higher visibility, and doctors can more conveniently and quickly calibrate the extreme point. When the intelligent recognition is used, compared with the image data and the two-dimensional image (data completion needs to be performed on the image data or the two-dimensional image in manners such as an interpolation algorithm to obtain the extreme point), the three-dimensional model has a continuous shape (in other words, a contour), and the extreme point can be quickly calculated without the data completion. Moreover, the calculation amount is small, the calculation speed is fast, the calculation efficiency is high, and less calculation resources are consumed.

In some possible implementations, the processor is configured to plan the path in the manners described below.

A three-dimensional model of the brain of the patient is generated according to image data.

The three-dimensional model is displayed by using a display device, where the three-dimensional model includes a three-dimensional structure of the nucleus accumbens and a three-dimensional structure of the anterior limb of the internal capsule.

A path planning operation is received by using an interaction device, and in response to the path planning operation, the path is planned.

In the three-dimensional model, both the nucleus accumbens and the anterior limb of the internal capsule can be displayed. Doctors design a surgical path according to three-dimensional structures of two displayed target sites (the nucleus accumbens and the anterior limb of the internal capsule).

In a second aspect, the present application provides a path planning method. The method includes the steps described below.

A path through which an electrode lead is implanted into an intracranial region of a patient is planned, where the planned path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient.

The electrode lead is configured to deliver electrical stimulation to the anterior limb of the internal capsule and the nucleus accumbens.

In some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

**In** some possible implementations, planning the path through which the electrode lead is implanted into the intracranial region of the patient includes the steps described below.

Image data of the brain of the patient is acquired, where the image data includes magnetic resonance image data and/or CT image data.

An extreme point of a ventral contour of the nucleus accumbens is positioned according to the image data to obtain positioning data of the extreme point.

The path through which the electrode lead is implanted into the intracranial region of the patient is planned according to the positioning data of the extreme point so that a straight line where the path is located passes through the extreme point from a superior side to an inferior side, from an anterior side to a posterior side and from a lateral side to a medial side.

**In** some possible implementations, a process of positioning the straight line includes the steps described below.

A set point of the anterior limb of the internal capsule is positioned according to the image data to obtain positioning data of the set point.

The straight line is positioned according to the positioning data of the set point and the positioning data of the extreme point so that the straight line passes through the set point and the extreme point.

**In** some possible implementations, positioning the set point of the anterior limb of the internal capsule according to the image data includes the steps described below.

A segmentation result of the anterior limb of the internal capsule is acquired according to the image data, where the segmentation result of the anterior limb of the internal capsule is configured to indicate multiple regions of the anterior limb of the internal capsule.

One of the multiple regions is determined as a target region according to a projection relationship between each of the multiple regions and a frontal lobe so that a white matter tract traveling in the target region can be projected into a target prefrontal cortex, where the target frontal cortex is a frontal cortex associated with the disease of the patient.

The set point of the anterior limb of the internal capsule is positioned from the target region.

In some possible implementations, a process of positioning the straight line includes the steps described below.

The straight line is enabled to be in a reference plane, and an acute included angle formed between the straight line and any transverse plane of the brain is enabled to be a second preset angle.

The reference plane satisfies the following conditions: the extreme point is in the reference plane, an acute included angle formed between the reference plane and any sagittal plane of the brain is a first preset angle, and a normal of the reference plane is parallel to any coronal plane of the brain.

In some possible implementations, the first preset angle is not less than 5 degrees and is not greater than 85 degrees, and the second preset angle is not less than 15 degrees and is less than 90 degrees.

In some possible implementations, the first preset angle is not less than 15 degrees and is not greater than 45 degrees, and the second preset angle is not less than 40 degrees and is not greater than 85 degrees.

In some possible implementations, a process of determining the first preset angle and the second preset angle includes the steps described below.

A set point of the anterior limb of the internal capsule is positioned according to the image data to obtain positioning data of the set point.

The first preset angle and the second preset angle are determined according to the positioning data of the set point and the positioning data of the extreme point.

In some possible implementations, positioning the extreme point of the ventral contour of the nucleus accumbens according to the image data includes the steps described below.

A reference point is positioned according to the image data, and a two-dimensional image of the brain corresponding to the reference point is generated, where the two-dimensional image of the brain corresponding to the reference point includes one or more of a two-dimensional image of a coronal plane, a two-dimensional image of a sagittal plane and a two-dimensional image of a transverse plane.

The extreme point of the ventral contour of the nucleus accumbens is positioned by using the two-dimensional image of the brain corresponding to the reference point.

In some possible implementations, the reference point is a midpoint of a connecting line between an anterior commissure and a posterior commissure of the brain.

Alternatively, the reference point is an anterior commissure of the brain.

Alternatively, the reference point is a posterior commissure of the brain.

In some possible implementations, positioning the extreme point of the ventral contour of the nucleus accumbens by using the two-dimensional image of the brain corresponding to the reference point includes the steps described below.

An adjustment operation for each two-dimensional image is received, and in response to the adjustment operation, one or more of the following parameters of the each two-dimensional image are adjusted: brightness, contrast, saturation, exposure, hue, grayscale and scaling.

The extreme point of the ventral contour of the nucleus accumbens is positioned by using the adjusted two-dimensional image.

In some possible implementations, the adjustment operation is an operation of adjusting a parametric curve and is configured to adjust the brightness, the contrast or the grayscale of the each two-dimensional image.

Alternatively, the adjustment operation is an operation of adjusting a parameter progress bar and is configured to adjust the brightness, the contrast, the saturation, the exposure, the hue, the grayscale and the scaling of the each two-dimensional image.

Alternatively, the adjustment operation is an operation of adjusting a parameter value and is configured to adjust the brightness, the contrast, the saturation, the exposure, the hue, the grayscale and the scaling of the each two-dimensional image.

In some possible implementations, positioning the extreme point of the ventral contour of the nucleus accumbens according to the image data includes the steps described below.

A three-dimensional model of the brain of the patient is generated according to the image data.

The extreme point of the ventral contour of the nucleus accumbens is positioned by using the three-dimensional model.

In some possible implementations, planning the path through which the electrode lead is implanted into the intracranial region of the patient includes the steps described below.

A three-dimensional model of the brain of the patient is generated according to image data.

The three-dimensional model is displayed by using a display device, where the three-dimensional model includes a three-dimensional structure of the nucleus accumbens and a three-dimensional structure of the anterior limb of the internal capsule.

A path planning operation is received by using an interaction device, and in response to the path planning operation, the path is planned.

In a third aspect, the present application provides a surgical system. The surgical system includes any one of the above path planning apparatuses and a surgical apparatus.

The path planning apparatus is configured to plan a path through which an electrode lead is implanted into an intracranial region of a patient.

The surgical apparatus is configured to perform a surgery of implanting the electrode lead into the intracranial region of the patient according to the path so that the electrode lead is in contact with an anterior limb of an internal capsule and a nucleus accumbens of one hemisphere of a brain of the patient.

The electrode lead is configured to deliver electrical stimulation to the anterior limb of the internal capsule and the nucleus accumbens.

The implantation surgery is performed by using the surgical apparatus, and the electrode lead is implanted into the intracranial region of the patient according to the planned path.

In some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

In a fourth aspect, the present application provides a computer-readable storage medium storing a computer program, where the computer program, when executed by a processor, implements functions of any one of the above path planning apparatuses or implements steps of any one of the above path planning methods.

### BRIEF DESCRIPTION OF DRAWINGS

The present application is described below in conjunction with the drawings and embodiments.
FIG. 1 is a block diagram of a surgical system according to an embodiment of the present application.
FIG. 2 is a flowchart of a path planning method according to an embodiment of the present application.
FIG. 3 is a flowchart of a planned path according to an embodiment of the present application.
FIG. 4 illustrates a portion of a two-dimensional image of a coronal plane of a brain according to an embodiment of the present application.
FIG. 5 is a flowchart for positioning an extreme point according to an embodiment of the present application.
FIG. 6 is another flowchart for positioning an extreme point according to an embodiment of the present application.
FIG. 7 is another flowchart for positioning an extreme point according to an embodiment of the present application.
FIG. 8 is another flowchart of a planned path according to an embodiment of the present application.
FIG. 9 is a structure diagram of a viewing angle of a coronal plane of a brain according to an embodiment of the present application.
FIG. 10 is a structure diagram of a viewing angle of a sagittal plane of a brain according to an embodiment of the present application.
FIG. 11 illustrates a diagram illustrating a relationship between spatial positions of a second preset angle and a third preset angle according to an embodiment of the present application.
FIG. 12 is a block diagram of a path planning apparatus according to an embodiment of the present application.
FIG. 13 is a structure diagram of a program product for implementing a path planning method according to an embodiment of the present application.
FIG. 14 is a structure diagram of an anterior limb of an internal capsule according to an embodiment of the present application.

### DETAILED DESCRIPTION

The technical solutions in the present application are described below in conjunction with the drawings and the embodiments of the present application. It is to be noted that under the premise of no conflict, the embodiments or the technical features described below may be arbitrarily combined to form a new embodiment.

In embodiments of the present application, "at least one" refers to one or more and "multiple" refers to two or more. "And/or" is used for describing an association between associated objects and indicates three relations, for example, "A and/or B" may indicate the presence of A alone, the presence of both A and B and the presence of B alone, where A and B may be singular or plural. The character "/" generally indicates an "or" relation between associated objects. "At least one of the following" or a similar expression thereof refers to any combination of items, including any combination of singular items or plural items. For example, at least one of a, b or c may indicate a, b, c, "a and b", "a and c", "b and c" or "a and b and c", where a, b and c may be singular or plural. It is to be noted that "at least one" may also be explained as "one or more".

It is to be further noted that in the embodiments of the present application, words such as "exemplary" or "for example" are used for indicating examples, illustrations or descriptions. Any embodiment or design solution described as "exemplary" or "for example" in the embodiments of the present application should not be construed as being more preferred or advantageous than other embodiments or design solutions. Specifically, the use of words such as "exemplary" or "for example" is intended to present relevant concepts in a specific manner.

Meanings of terms such as superior, inferior, anterior, posterior, lateral and medial in the embodiments of the present application are anatomical concepts. Human anatomical orientations are standard terms in anatomy for describing relative positions and motion directions of human organs, and a standard anatomical posture of placing both hands on two sides of the body and with palms facing anteriorly is used as a standard.

Some anatomical terms are listed below, most of which appear hereinafter.

Human anatomical section: sagittal plane, transverse plane (also known as horizontal plane) and coronal plane.

In human anatomy, the following orientation terms have the meanings described below.

Superior: near the head. The superior side is also referred to as the proximal side when describing the limbs. The superior side is also referred to as the dorsal side when describing the foot.

Inferior: near the foot. The inferior side is also referred to as the distal side when describing the limbs. The inferior side is also referred to as the plantar side when describing the foot.

Anterior: near the abdomen. The anterior side is also referred to as the volar side when describing the palm.

Posterior: near the back. The posterior side is also referred to as the dorsal side when describing the palm.

Medial side: near the middle sagittal plane. The medial side is also referred to as the ulnar side when describing the forearm and is also referred to as the tibial side when describing the lower leg.

Lateral side: away from the middle sagittal plane. The lateral side is also referred to as the radial side when describing the forearm and is also referred to as the fibular side when describing the lower leg.

Proximal side or proximal end: the parts of the limbs near the trunk.

Distal side or distal end: the parts of the limbs away from the trunk.

Local organs are also described by the terms described below.

Medial: near the inner cavity.

Lateral: away from the inner cavity.

Superficial: near the body or the surface.

Deep: away from the body or the surface.

One of the application fields (an implantable device) in the embodiments of the present application is briefly described below.

An implantable neurostimulation system (an implantable medical system) mainly includes a stimulator implanted into a patient and a program control device disposed in the patient *in vitro.* In an existing neuromodulation technology, an electrode is mainly implanted into a particular structure (a target site) *in vivo* through a stereotactic surgery, and an electrical pulse is sent by the stimulator implanted into the patient to the target site via the electrode to regulate electrical activity and functions of a corresponding neural structure and a network, thereby improving a symptom and alleviating a disease pain. The stimulator may be any one of an implantable nerve electrical stimulation apparatus, an implantable cardiac electrical stimulation system (also referred to as a cardiac pacemaker), an implantable drug delivery system (IDDS) and a lead switching apparatus. The implanted nerve electrical stimulation apparatus is, for example, a deep brain stimulation (DBS) system, an implantable cortical nerve stimulation (CNS) system, an implantable spinal cord stimulation (SCS) system, an implantable sacral nerve stimulation (SNS) system or an implantable vagus nerve stimulation (VNS) system.

The stimulator may include an IPG, an extended lead and an electrode lead. Disposed in the patient *in vivo* and in response to a program control instruction sent by the program control device, the implantable pulse generator (IPG) provides controllable electrical stimulation energy to an internal tissue depending on a sealed cell and a circuit and delivers one or two controllable particular electrical stimulations to a particular region of the internal tissue via the implanted extended lead and electrode lead. The extended lead is used together with the IPG. As a transmission medium of an electrical stimulation signal, the extended lead transmits the electrical stimulation signal generated by the IPG to the electrode lead. The electrode lead delivers electrical stimulation to the particular region of the internal tissue via multiple electrode contacts. One or more electrode leads are disposed on one side or two sides of the stimulator. Multiple electrode contacts are disposed on the electrode lead and may be evenly or non-evenly arranged in a circumferential direction of the electrode lead. As an example, the electrode contacts may be arranged in the circumferential direction of the electrode lead in an array of four rows and three columns (12 electrode contacts in total). The electrode contacts may include stimulation electrode contacts and/or acquisition electrode contacts. The electrode contact may be, for example, in a shape of a sheet, a ring or a dot.

In some possible embodiments, the stimulated internal tissue may be a brain tissue of the patient, and the stimulated part may be a particular part of the brain tissue. When disease types of patients are different, stimulated parts are generally different, and the number of used stimulation contacts (single source or multi-source), the application of one or more (single channel or multi-channel) particular electrical stimulation signals and stimulation parameter data are also different. The applicable disease types are not limited in the embodiments of the present application and may be disease types applicable to deep brain stimulation (DBS), spinal cord stimulation (SCS), pelvic stimulation, gastric stimulation, periphery nerve stimulation and functional electrical stimulation. Disease types that may be treated or managed by DBS include, but are not limited to, seizure disorders (for example, epilepsy), pain, migraine, mental illnesses (for example, major depressive disorder (MDD)), bipolar disorder, anxiety disorder, post-traumatic stress disorder, dysthymia, obsessive-compulsive disorder (OCD), behavior disorder, mood disorder, memory disorder, mental state disorder, movement disorders (for example, essential tremor or Parkinson's disease), Huntington's disease, Alzheimer's disease, drug addiction, autism or other neurological or psychiatric disorders and impairment.

In the embodiments of the present application, when a program control connection is established between the program control device and the stimulator, the program control device may be used for adjusting stimulation parameters of the stimulator (different stimulation parameters correspond to different electrical stimulation signals), or the stimulator may sense an electrophysiological activity of a deep brain of the patient to acquire an electrophysiological signal, and the adjustment of the stimulation parameters of the stimulator may be continued by using the acquired electrophysiological signal.

The stimulation parameter may include at least one of a stimulation contact identifier (for example, may be a 2# electrode contact or a 3# electrode contact), a frequency (for example, the number of electrical stimulation pulse signals within a unit time of 1 s, in Hz), a pulse width (the duration of each pulse, in µs), an amplitude (generally represented by voltage, that is, the strength of each pulse, in V), a timing (for example, may be continuation or burst, where burst refers to a discontinuous timing behavior formed by multiple processes), a stimulation mode (including one or more of a current mode, a voltage mode, a timing stimulation mode and a cyclic stimulation mode), doctor-controlled upper and lower limits (a range that can be adjusted by a doctor) and patient-controlled upper and lower limits (a range that can be autonomously adjusted by a patient).

In a specific application scenario, each stimulation parameter of the stimulator may be adjusted in the current mode or the voltage mode.

The program control device may be a doctor program control device (a program control device used by the doctor) or a patient program control device (a program control device used by the patient). The doctor program control device may be, for example, an intelligent terminal device equipped with program control software, such as a tablet computer, a notebook computer, a desktop computer or a mobile phone. The patient program control device may be, for example, an intelligent terminal device equipped with program control software, such as a tablet computer, a notebook computer, a desktop computer or a mobile phone, and may also be another electronic device (for example, a charger or data acquisition device with a program control function) with a program control function.

The data interaction between the doctor program control device and the stimulator is not limited in the embodiments of the present application. When the doctor performs remote program control, the doctor program control device can perform data interaction with the stimulator via a server and the patient program control device. When the doctor performs offline program control with the patient face to face, the doctor program control device can perform data interaction with the stimulator via the patient program control device and can also directly perform data interaction with the stimulator.

In some optional embodiments, the patient program control device may include a host (in communication with the server) and a slave (in communication with the stimulator), and the host can be communicatively connected to the slave. The doctor program control device can perform data interaction with the server via a 3G/4G/5G network. The server can perform data interaction with the host via the 3G/4G/5G network. The host can perform data interaction with the slave via a Bluetooth protocol/WIFI protocol/USB protocol. The slave can perform data interaction with the stimulator via an operating frequency band of 401-406 MHz/operating frequency band of 2.4-2.48 GHz. The doctor program control device can directly perform data interaction with the stimulator via the operating frequency band of 401-406 MHz/operating frequency band of 2.4-2.48 GHz.

An invention background of the embodiments of the present application is described below.

Drug abuse and dependence are important social problems facing the world. Therefore, countries generally have high demand for drug addiction treatment. The basic treatment of drug addiction includes two aspects: detoxification and prevention of relapse. Currently, existing therapies have a better detoxification effect but can do nothing about psychological dependence caused by drugs, which does not effectively prevent relapse. Patients tend to fall into a vicious cycle of "taking drugs-detoxification-relapse". The key to preventing relapse lies in whether the drug addicts' craving for drugs can be effectively inhibited.

According to the researches of the inventors, the reward circuit of the midbrain-limbic-cortical system is closely associated with addictive behavior, depressive disorder and obsessive-compulsive disorder. According to years of clinical experience in surgical intervention in addiction and domestic and international research advances in addiction, the inventors propose the theory of relapse prevention of drug addiction that joint electrical stimulation is performed on a nucleus accumbens and an anterior limb of an internal capsule. Image positioning of the two target sites (the nucleus accumbens and the anterior limb of the internal capsule) and surgical planning for the implantation path of the stimulation electrode are core premises of the treatment.

There is one anterior limb of the internal capsule on each of the left and right sides of the human brain, traveling between the lateral putamen and the medial caudate nucleus with the nucleus accumbens on the ventromedial side. The anterior limb of the internal capsule consists of ascending and descending white matter tracts, joining to the frontal lobe, striatum, thalamus, brainstem, cerebellum and spinal cord to participate in the occurrence and development of human emotion, cognition, decision-making and psychiatric disorder diseases. The anterior limb of the internal capsule is one of the target sites of destruction surgeries for treating psychiatric disorder diseases, and the region that the white matter tracts traveling in the anterior limb of the internal capsule project to the frontal lobe is correlated with the pattern of arrangement from the ventral side to the dorsal side and from the medial side to the lateral side in the anterior limb of the internal capsule. The segmentation and refinement of the anterior limb of the internal capsule are the premises for improving the efficacy of DBS in treating psychiatric disorder diseases.

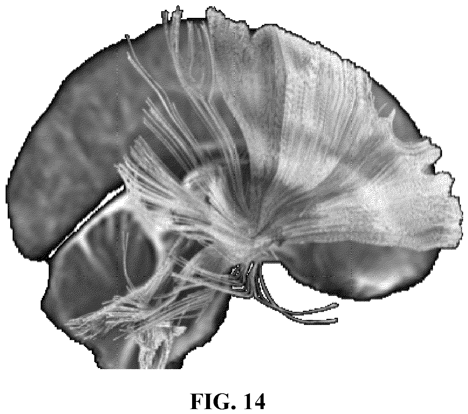

As shown in FIG. 14, the anterior limb of the internal capsule may be segmented into multiple regions (for example, 2, 3, 4, 6, 12, 18 or 24). White matters traveling in different regions are projected to different regions of the frontal lobe. Different prefrontal cortex regions participate in different human functions and symptoms of mental illnesses.

Hereinafter, the planning steps and paths of the intervention sites of the nucleus accumbens and the anterior limb of the internal capsule are defined in the embodiments of the present application according to brain imaging.

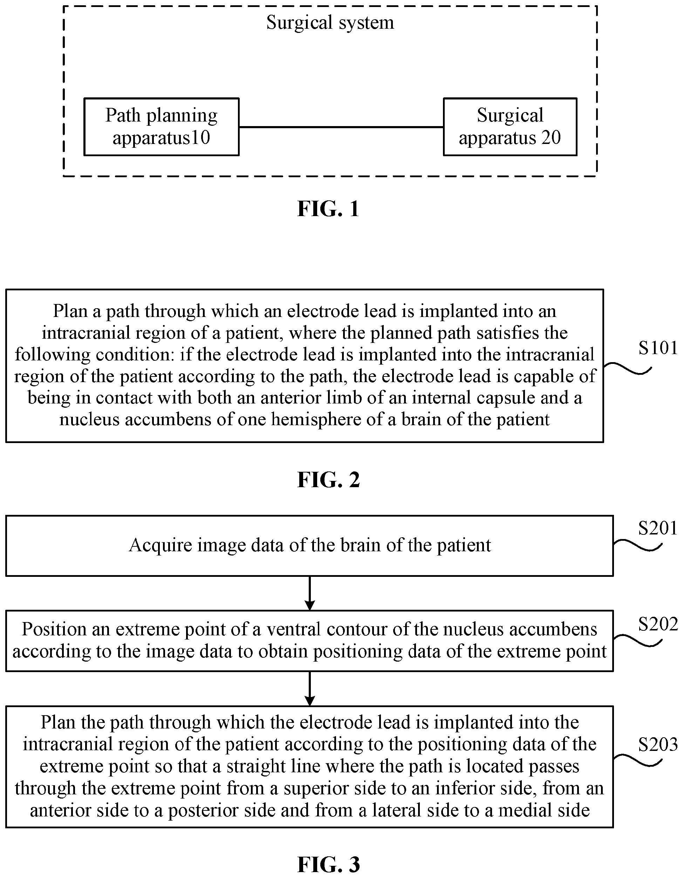

Referring to FIG. 1, FIG. 1 is a block diagram of a surgical system according to an embodiment of the present application.

The surgical system includes a path planning apparatus 10 and a surgical apparatus 20.

The path planning apparatus 10 is configured to plan a path through which an electrode lead is implanted into an intracranial region of a patient.

The surgical apparatus 20 is configured to perform a surgery of implanting the electrode lead into the intracranial region of the patient according to the path so that the electrode lead is in contact with an anterior limb of an internal capsule and a nucleus accumbens of one hemisphere of a brain of the patient.

The electrode lead is configured to deliver electrical stimulation to the anterior limb of the internal capsule and the nucleus accumbens.

**In** this manner, the implantation surgery is performed by using the surgical apparatus 20, and the electrode lead is implanted into the intracranial region of the patient according to the planned path.

**In** some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

The surgical apparatus 20 is not limited in the embodiment of the present application and may include, for example, one or more of a surgical guide assembly, a drive assembly, a patient fixing assembly and an operating table. A manner in which the surgical apparatus 20 performs the surgery is not limited in the embodiment of the present application. The surgical apparatus 20 may automatically perform surgical steps according to a preset surgical plan, or may be controlled by a doctor to perform the surgical steps.

In some possible implementations, the surgical system may further include a medical imaging device configured to acquire image data of the patient. Here, the image data refers to medical image data. The medical imaging device may include, for example, one or more of a CT device, an MR device, a PET device, a PET-CT device and a PET-MR device. Correspondingly, the image data may include, for example, one or more of CT image data, MR image data, PET image data, PET-CT image data and PET-MR image data. CT is computed tomography, MR is magnetic resonance, and PET is positron emission tomography.

In some possible implementations, the path planning apparatus 10 may be integrated with the surgical apparatus 20.

In the embodiment of the present application, the path planning apparatus 10 may be configured to implement steps of a path planning method. The path planning method is described hereinafter.

Referring to FIG. 2, FIG. 2 is a flowchart of a path planning method according to an embodiment of the present application.

The method includes the steps described below.

In step S101, a path through which an electrode lead is implanted into an intracranial region of a patient is planned, where the planned path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient.

The electrode lead is configured to deliver electrical stimulation to the anterior limb of the internal capsule and the nucleus accumbens.

In this manner, an implantation path of a particular electrode lead is planned, and the electrode lead is implanted into one hemisphere of the brain of the patient (for example, a left hemisphere or a right hemisphere) according to the path so that joint electrical stimulation can be performed on a nucleus accumbens and an anterior limb of an internal capsule of this hemisphere to treat the patient.

The path planned by the path planning method enables the electrode lead to be in contact with an anterior limb of an internal capsule and a nucleus accumbens of one hemisphere. In this manner, the electrode lead can simultaneously deliver electrical stimulation to the anterior limb of the internal capsule and the nucleus accumbens, and a joint electrical stimulation therapy is achieved.

In a DBS field, a doctor performed an electrical stimulation therapy on only one particular brain tissue in the past. Therefore, after one electrode lead was implanted into an intracranial region of a patient, even though multiple electrode patches may be on the electrode lead, these electrode patches were used for delivering electrical stimulation to the same brain tissue (nucleus) and cannot simultaneously deliver electrical stimulation to multiple brain tissues, and no joint electrical stimulation therapy was performed on two particular brain tissues (an anterior limb of an internal capsule and a nucleus accumbens).

The path planning method provided in the embodiment of the present application can plan the implantation path of the electrode lead so that after the electrode lead is implanted into the intracranial region of the patient, a joint electrical stimulation therapy can be performed on an anterior limb of an internal capsule and a nucleus accumbens of one hemisphere of the brain, having a relatively good therapeutical effect on diseases such as addictive behavior, depressive disorder and obsessive-compulsive disorder.

In some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

In this manner, the electrode lead passes through the anterior limb of the internal capsule and the nucleus accumbens in a particular direction, and the joint electrical stimulation for the anterior limb of the internal capsule and the nucleus accumbens is used for the treatment of a particular type of disease, for example, the treatment of multiple mental disorders, thereby effectively alleviating a condition of the patient or even curing a disease of the patient.

One or more electrode leads can be implanted into each hemisphere of the brain according to the above path.

Referring to FIG. 3, FIG. 3 is a flowchart of a planned path according to an embodiment of the present application. In some possible implementations, step S101 may include the steps described below.

In step S201, image data of the brain of the patient is acquired.

In step S202, an extreme point of a ventral contour of the nucleus accumbens is positioned according to the image data to obtain positioning data of the extreme point.

In step S203, the path through which the electrode lead is implanted into the intracranial region of the patient is planned according to the positioning data of the extreme point so that a straight line where the path is located passes through the extreme point from a superior side to an inferior side, from an anterior side to a posterior side and from a lateral side to a medial side.

In this manner, the image data is used for positioning the extreme point of the ventral contour of the nucleus accumbens, and the extreme point is used for planning the implantation path of the electrode lead so that the straight line where the planned path is located passes through the extreme point.

In some possible implementations, a process of positioning the straight line includes the steps described below.

A set point of the anterior limb of the internal capsule is positioned according to the image data to obtain positioning data of the set point.

The straight line is positioned according to the positioning data of the set point and the positioning data of the extreme point so that the straight line passes through the set point and the extreme point.

In this manner, the set point of the anterior limb of the internal capsule and the extreme point of the nucleus accumbens are used for forming a constraint on the path, and the implantation path of the electrode lead is planned in a two-point-one-line manner so that the electrode lead sequentially passes through the anterior limb of the internal capsule and the nucleus accumbens in an implantation process from a superior side to an inferior side, from an anterior side to a posterior side and from a lateral side to a medial side.

Specifically, a projection relationship between each region and a frontal lobe is determined according to the segmentation of the anterior limb of the internal capsule to determine a specific coordinate point used when DBS interferes with the anterior limb of the internal capsule, thereby planning the electrode path with the nucleus accumbens and the anterior limb of the internal capsule in the two-point-one-line manner.

When the anterior limb of the internal capsule (the target site) is defined, according to an individual difference of a symptom of the patient, the target site may be set as follows: a white matter tract traveling in the region is projected into a particular prefrontal cortex associated with the symptom of the patient.

In some possible implementations, positioning the set point of the anterior limb of the internal capsule according to the image data includes the steps described below.

A segmentation result of the anterior limb of the internal capsule is acquired according to the image data, where the segmentation result of the anterior limb of the internal capsule is configured to indicate multiple regions of the anterior limb of the internal capsule.

One region is determined (from the multiple regions) as a target region according to a projection relationship between each region and a frontal lobe so that a white matter tract traveling in the target region can be projected into a target prefrontal cortex, where the target frontal cortex is a frontal cortex associated with the disease of the patient.

The set point of the anterior limb of the internal capsule is positioned from the target region.

In this manner, the image data is used for acquiring the segmentation result of the anterior limb of the internal capsule, and the segmentation result of the anterior limb of the internal capsule is used for positioning the set point of the anterior limb of the internal capsule. When the implantation path passes through the set point, the electrode lead can release electrical stimulation energy to a contact portion (the target region) of the anterior limb of the internal capsule with the electrode lead. This energy is projected into the target prefrontal cortex via the white matter tract in the target region and accurately acts on a particular prefrontal cortex associated with the disease of the patient, thereby achieving the precise treatment of a mental disorder.

In some possible implementations, a process of positioning the straight line includes the steps described below.

The straight line is enabled to be in a reference plane, and an acute included angle formed between the straight line and any transverse plane of the brain is enabled to be a second preset angle.

The reference plane satisfies the following conditions: the extreme point is in the reference plane, an acute included angle formed between the reference plane and any sagittal plane of the brain is a first preset angle, and a normal of the reference plane is parallel to any coronal plane of the brain.

In mathematics, extremum is a general term of maximum and minimum and refers to a function value at a point where a function reaches a maximum value or a minimum value on a domain. The point where the function reaches the extremum is referred to as an extreme point. This domain may be a neighborhood or an entire function domain (in this case, the extremum is referred to as an extremum value, a global extremum or an absolute extremum).

The positioning data is not limited in the embodiment of the present application and may be, for example, coordinate data. A coordinate system used may be, for example, a Cartesian coordinate system, a planar polar coordinate system, a cylindrical coordinate system (or referred to as a cylinder coordinate system) or a spherical coordinate system (or referred to as a sphere coordinate system). In practical application, one or more coordinate systems can be established according to a requirement of a doctor for a surgery, and a mapping relationship between multiple coordinate systems can be established to perform the surgery.

In this manner, the straight line where the path is located is located in a particular reference plane, and a particular acute included angle (the second preset angle) is formed between the straight line and any transverse plane of the brain.

The setting of the reference plane needs to satisfy multiple conditions. Not only the extreme point is set in the reference plane, but also a particular acute included angle (the first preset angle) is formed between the reference plane and any sagittal plane, and the normal of the reference plane is parallel to the coronal plane, that is, a projection of the reference plane on the coronal plane is a straight line.

In this manner, the straight line where the planned path is located can pass through the anterior limb of the internal capsule and the nucleus accumbens in a particular direction and at a particular angle. Therefore, the electrode lead implanted according to such a path can pass through the anterior limb of the internal capsule and the nucleus accumbens in the particular direction and at the particular angle, thereby performing the joint electrical stimulation therapy on the two brain tissues.

In some possible implementations, the first preset angle is not less than 5 degrees and is not greater than 85 degrees, and the second preset angle is not less than 15 degrees and is less than 90 degrees.

In this manner, since a brain structure varies with each individual and a brain structure of each person is different, the first preset angle and the second preset angle may be values within a relatively wide value range, provided that joint electrical stimulation can be performed on an anterior limb of an internal capsule and a nucleus accumbens of one hemisphere of the brain of the patient.

In some possible implementations, the first preset angle is not less than 15 degrees and is not greater than 45 degrees, and the second preset angle is not less than 40 degrees and is not greater than 85 degrees.

In this manner, although a brain structure of each person is different, brain structures of most people are relatively similar. In terms of probability, a more accurate angle range can be preferentially used to quickly determine a direction of the implantation path, which is sufficient to cope with a general situation of most people. For a very small number of people whose brain structures are very different from those of ordinary people, a wider range can be enabled as an alternative to help a doctor plan a path for these few people to implant electrode leads.

In some possible implementations, a process of determining the first preset angle and the second preset angle includes the steps described below.

A set point of the anterior limb of the internal capsule is positioned according to the image data to obtain positioning data of the set point.

The first preset angle and the second preset angle are determined according to the positioning data of the set point and the positioning data of the extreme point.

In this manner, before the first preset angle, the second preset angle and the extreme point are used for determining the implantation path, the set point of the anterior limb of the internal capsule and the extreme point of the nucleus accumbens are used as the constraint, the value range of the first preset angle and the second preset angle are significantly narrowed, and a range where the implantation path of the electrode lead is located in the implantation process is significantly narrowed. In particular, when the first preset angle and the second preset angle may be values within a relatively wide value range, an amount of data for calculating all feasible solutions of the first preset angle and the second preset angle can be significantly reduced.

In some possible implementations, a process of determining the first preset angle and the second preset angle includes the steps described below.

A segmentation result of the anterior limb of the internal capsule is acquired according to the image data, where the segmentation result of the anterior limb of the internal capsule is configured to indicate multiple regions of the anterior limb of the internal capsule.

One region is determined (from the multiple regions) as a target region according to a projection relationship between each region and a frontal lobe to obtain positioning data of the target region so that a white matter tract traveling in the target region can be projected into a target prefrontal cortex, where the target frontal cortex is a frontal cortex associated with the disease of the patient.

The first preset angle and the second preset angle are determined according to the positioning data of the target region and the positioning data of the extreme point.

In some other possible implementations, step S101 may include the steps described below.

Image data of the brain of the patient is acquired.

Positioning data of the anterior limb of the internal capsule and positioning data of the nucleus accumbens are acquired according to the image data.

The path through which the electrode lead is implanted into the intracranial region of the patient is planned according to the positioning data of the anterior limb of the internal capsule and the positioning data of the nucleus accumbens so that a straight line where the path is located passes through the anterior limb of the internal capsule and the nucleus accumbens from a superior side to an inferior side, from an anterior side to a posterior side and from a lateral side to a medial side.

In some other possible implementations, step S101 may include the steps described below.

Image data of the brain of the patient is acquired.

Contour data of the anterior limb of the internal capsule and contour data of the nucleus accumbens are acquired according to the image data.

The contour data of the anterior limb of the internal capsule and the contour data of the nucleus accumbens are used for forming a space constraint on the path, and the path through which the electrode lead is implanted into the intracranial region of the patient is planned according to the poisoning data of the extreme point so that a straight line where the path is located passes through the anterior limb of the internal capsule and the nucleus accumbens from a superior side to an inferior side, from an anterior side to a posterior side and from a lateral side to a medial side.

In some other possible implementations, step S101 may include the steps described below.

Image data of the brain of the patient is acquired.

Contour data of the anterior limb of the internal capsule, contour data of the nucleus accumbens and contour data of a caudate nucleus, a putamen and a medial diagonal band that are located in the same hemisphere as the anterior limb of the internal capsule and the nucleus accumbens are acquired according to the image data.

The contour data of the anterior limb of the internal capsule, the contour data of the nucleus accumbens, the contour data of the caudate nucleus, the contour data of the putamen and the contour data of the medial diagonal band are used for forming a space constraint on the path, and the path through which the electrode lead is implanted into the intracranial region of the patient is planned so that a straight line where the path is located passes through the anterior limb of the internal capsule and the nucleus accumbens from a superior side to an inferior side, from an anterior side to a posterior side and from a lateral side to a medial side.

In some other possible implementations, step S101 may include the steps described below.

Image data of the brain of the patient is acquired.

A three-dimensional model of the brain of the patient is acquired according to image data.

In the three-dimensional model, the contour of the anterior limb of the internal capsule and the contour of the nucleus accumbens are used for forming a space constraint on the path, and the path through which the electrode lead is implanted into the intracranial region of the patient is planned so that a straight line where the path is located passes through the anterior limb of the internal capsule and the nucleus accumbens from a superior side to an inferior side, from an anterior side to a posterior side and from a lateral side to a medial side.

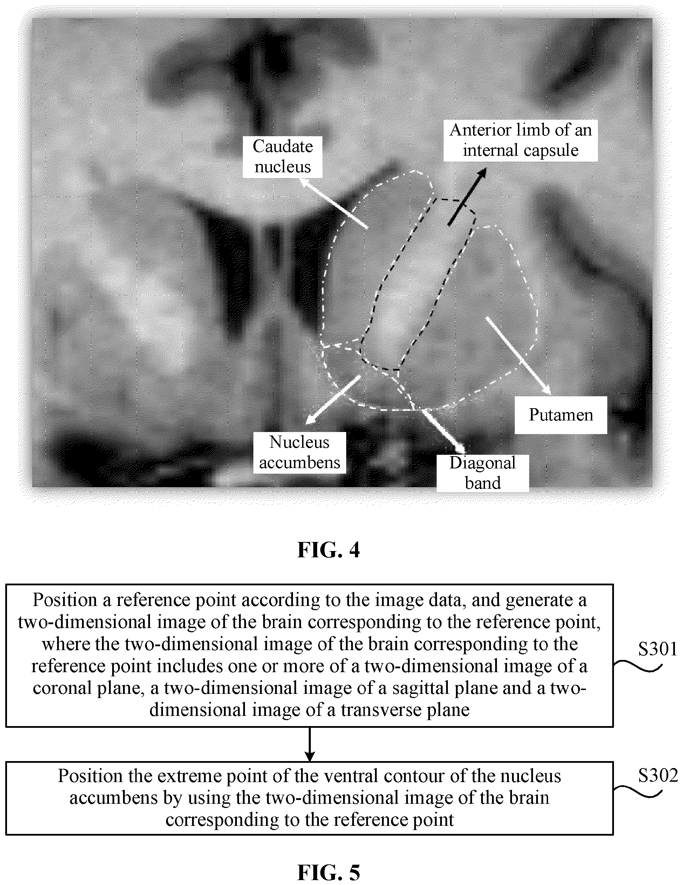

Referring to FIGS. 4 and 5, FIG. 4 illustrates a portion of a two-dimensional image of a coronal plane of a brain according to an embodiment of the present application, and FIG. 5 is a flowchart for positioning an extreme point according to an embodiment of the present application. Positions of a caudate nucleus, a putamen, a nucleus accumbens, an anterior limb of an internal capsule and a medial diagonal band are illustrated in FIG. 4.

In some possible implementations, step S202 may include the steps described below.

In step S301, a reference point is positioned according to the image data, and a two-dimensional image of the brain corresponding to the reference point is generated, where the two-dimensional image of the brain corresponding to the reference point includes one or more of a two-dimensional image of a coronal plane, a two-dimensional image of a sagittal plane and a two-dimensional image of a transverse plane.

In step S302, the extreme point of the ventral contour of the nucleus accumbens is positioned by using the two-dimensional image of the brain corresponding to the reference point.

In this manner, the image data is used for positioning the reference point. The reference point may be, for example, an anterior commissure of the brain, a posterior commissure of the brain or a midpoint of the anterior commissure and the posterior commissure of the brain. After the reference point is positioned, a coronal plane, a sagittal plane and a transverse plane of the brain corresponding to the reference point can be positioned, that is, a coronal plane passing through the reference point (in other words, the reference point is in the coronal plane), a sagittal plane passing through the reference point and a transverse plane passing through the reference point, and three-dimensional image data is used for generating one or more of the two-dimensional images corresponding to the coronal plane, the sagittal plane and the transverse plane. Since the ventral contour of the nucleus accumbens presents a parabola-like shape in the above two-dimensional image (an opening of the parabola is upward and outward), the extreme point (a vertex of the parabola) of the ventral contour of the nucleus accumbens can be positioned from the parabola. Moreover, the step of positioning the extreme point may be achieved in an intelligent recognition or manual calibration manner.

For doctors, the manner for positioning the extreme point is very intuitive, and the extreme point can be quickly calibrated according to experience.

In the intelligent recognition manner, the contour of the nucleus accumbens can be quickly recognized through an edge detection algorithm and/or an image segmentation algorithm, thereby positioning the extreme point of the ventral contour of the nucleus accumbens. Both an algorithm model corresponding to the edge detection algorithm and an algorithm model corresponding to the image segmentation algorithm may be pre-trained algorithm models. These trained algorithm models may be obtained through training in a supervised learning manner, may be obtained through training in a semi-supervised learning manner, or may be obtained through training in an unsupervised learning manner.

The path planning method provided in the embodiment of the present application generates the two-dimensional image by using the reference point and positions the extreme point by using the two-dimensional image. This manner of positioning the extreme point has a simple calculation process, a small calculation amount and a fast calculation speed and consumes less calculation resources.

The parabola is an axisymmetric graphic whose symmetry axis is simply called an axis. An intersection point of the parabola with the axis of the parabola is called the vertex of the parabola.

In some possible implementations, the reference point is a midpoint of a connecting line between an anterior commissure and a posterior commissure of the brain.

Alternatively, the reference point is an anterior commissure of the brain.

Alternatively, the reference point is a posterior commissure of the brain.

In this manner, using the anterior commissure of the brain, the posterior commissure of the brain or the midpoint of the connecting line between the anterior commissure and the posterior commissure of the brain as the reference point has the advantages of simple operation and easy implementation in the intelligent recognition manner or the manual calibration manner. For doctors, the manner for positioning the reference point is very intuitive, and the reference point can be quickly calibrated according to experience. In the intelligent recognition manner, a contour of an entire brain can be quickly obtained through the edge detection algorithm, and the anterior commissure and the posterior commissure are positioned from the contour of the entire brain, thereby positioning the reference point.

In the embodiment of the present application, anterior commissure has two translation versions: anterior commissure and precommissure, which have the same meaning. Similarly, posterior commissure has two translation versions: posterior commissure and epithalamic commissure, which have the same meaning.

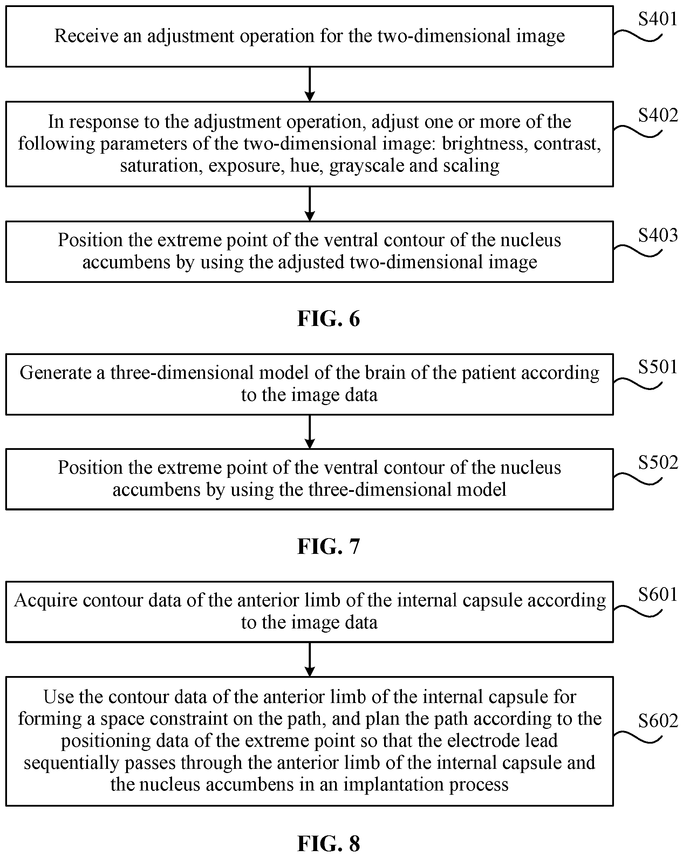

Referring to FIG. 6, FIG. 6 is another flowchart for positioning an extreme point according to an embodiment of the present application.

In some possible implementations, step S302 may include the steps described below.

In step S401, an adjustment operation for each two-dimensional image is received.

In step S402, in response to the adjustment operation, one or more of the following parameters of the each two-dimensional image are adjusted: brightness, contrast, saturation, exposure, hue, grayscale and scaling.

In step S403, the extreme point of the ventral contour of the nucleus accumbens is positioned by using the adjusted two-dimensional image.

In this manner, doctors can manually adjust the parameter of the two-dimensional image. Adjusting the parameter can change a display effect of the two-dimensional image, thereby facilitating determining the extreme point from the two-dimensional image.

The adjusted parameter is not limited in the embodiment of the present application and may be, for example, one or more of multiple parameters that affect the display effect.

Apparently, the adjustment to one or more of these parameters can make the ventral contour of the nucleus accumbens more convex and clearer (in other words, the contrast is higher) so that the positioned extreme point is more accurate and reliable, the positioning speed is faster and the positioning efficiency is high, thereby improving overall path planning efficiency.

An object of adjusting the parameter in this manner is to presents a boundary and a contour (including a ventral side, a dorsal side, a medial side, a lateral side, an anterior side and a posterior side) of the nucleus accumbens more clearly and converts an incompletely visible contour before the adjustment into a relatively clear contour after the adjustment, thereby facilitating positioning the ventral extreme point (the extreme point of the ventral contour of the nucleus accumbens).

In some possible implementations, step S401 may include: displaying the two-dimensional image by using an interaction device, and receiving the adjustment operation for the two-dimensional image by using the interaction device.

In some possible implementations, the interaction device has a display assembly and a touch assembly. The interaction device may be, for example, a mobile phone, a tablet computer, a notebook computer, a desktop computer or another intelligent terminal device.

In some possible implementations, the adjustment operation is an operation of adjusting a parametric curve and is configured to adjust the brightness, the contrast or the grayscale of the each two-dimensional image. Alternatively, the adjustment operation is an operation of adjusting a parameter progress bar and is configured to adjust the brightness, the contrast, the saturation, the exposure, the hue, the grayscale and the scaling of the each two-dimensional image.

Alternatively, the adjustment operation is an operation of adjusting a parameter value and is configured to adjust the brightness, the contrast, the saturation, the exposure, the hue, the grayscale and the scaling of the each two-dimensional image.

In this manner, doctors are allowed to adjust the brightness or the contrast in a manner of adjusting the parametric curve, the doctors are allowed to adjust multiple parameters in a manner of adjusting the parameter progress bar, or the doctors are allowed to adjust the multiple parameters in a manner of adjusting the parameter value so that the doctors can select a manner of adjusting the parameters according to preferences of the doctors to quickly adjust the display effect of the two-dimensional image, thereby further improving the path planning efficiency.

When the adjustment operation is the operation of adjusting the parametric curve, one or more of brightness, contrast and grayscale of multiple regions in the two-dimensional image can be differently adjusted to highlight a contour region of the nucleus accumbens in the two-dimensional image. That is, in some possible implementations, brightness may be increased in some regions and decreased in other regions, and/or contrast may be increased in some regions and decreased in other regions, and/or grayscale may be increased in some regions and decreased in other regions.

Referring to FIG. 7, FIG. 7 is another flowchart for positioning an extreme point according to an embodiment of the present application. **In** some other possible implementations, step S202 may include the steps described below.

In step S501, a three-dimensional model of the brain of the patient is generated according to the image data.

In step S502, the extreme point of the ventral contour of the nucleus accumbens is positioned by using the three-dimensional model.

**In** this manner, the image data is used for generating the three-dimensional model of the brain, and the three-dimensional model is used for positioning the extremum point. The manner of positioning the extreme point may be intelligent recognition or manual calibration.

When the manual calibration is used, compared with the image data and the two-dimensional image, the three-dimensional model has higher visibility, and doctors can more conveniently and quickly calibrate the extreme point.

When the intelligent recognition is used, compared with the image data and the two-dimensional image (data completion generally needs to be performed on the image data or the two-dimensional image in manners such as an interpolation algorithm to obtain the extreme point), the three-dimensional model has a continuous shape (in other words, a contour), and the extreme point can be quickly calculated without the data completion. Moreover, the calculation amount is small, the calculation speed is fast, the calculation efficiency is high, and less calculation resources are consumed.

In some other possible implementations, step S101 may include the steps described below.

A three-dimensional model of the brain of the patient is generated according to image data.

The three-dimensional model is displayed by using a display device, where the three-dimensional model includes a three-dimensional structure of the nucleus accumbens and a three-dimensional structure of the anterior limb of the internal capsule.

A path planning operation is received by using an interaction device, and in response to the path planning operation, the path is planned.

In this manner, in the three-dimensional model, both the nucleus accumbens and the anterior limb of the internal capsule can be displayed. Doctors design a surgical path according to three-dimensional structures of two displayed target sites (the nucleus accumbens and the anterior limb of the internal capsule).

The interaction device is not limited in the embodiment of the present application. The interaction device may be, for example, a mobile phone, a tablet computer, a notebook computer, a desktop computer, a smart wearable device or another intelligent terminal device, or may be a console or a workstation.

A manner of receiving various (manual) operations by using the interaction device is not limited in the embodiment of the present application. The operations are divided according to input manners and may include, for example, a text input operation, an audio input operation, a video input operation, a button operation, a knob operation, a mouse operation, a keyboard operation and an intelligent stylus operation.

The above display device and interaction device may be integrated. For example, a touch display device may be used, that is, a tablet computer, a smart screen, a smartphone and a notebook computer with a touch screen.

Referring to FIG. 8, FIG. 8 is another flowchart of a planned path according to an embodiment of the present application. In some other possible implementations, step S202 may include the steps described below.

In step S601, contour data of the anterior limb of the internal capsule is acquired according to the image data.

In step S602, the contour data of the anterior limb of the internal capsule is used for forming a space constraint on the path, and the path is planned according to the positioning data of the extreme point so that the electrode lead sequentially passes through the anterior limb of the internal capsule and the nucleus accumbens in an implantation process.

In this manner, the image data is used for acquiring the contour data of the anterior limb of the internal capsule, and the contour data of the anterior limb of the internal capsule is used for forming the constraint on the path. In conjunction with the positioning data of the extreme point, the implantation path of the electrode lead is planned so that the electrode lead sequentially passes through the anterior limb of the internal capsule and the nucleus accumbens in the implantation process from a superior side to an inferior side, from an anterior side to a posterior side and from a lateral side to a medial side.

That is, before the first preset angle, the second preset angle and the extreme point are used for determining the implantation path, the contour of the anterior limb of the internal capsule is used as the constraint, and a range where the implantation path of the electrode lead is located in the implantation process is significantly narrowed. In particular, when the first preset angle and the second preset angle may be values within a relatively wide value range, an amount of data for calculating all feasible solutions of the first preset angle and the second preset angle can be significantly reduced.

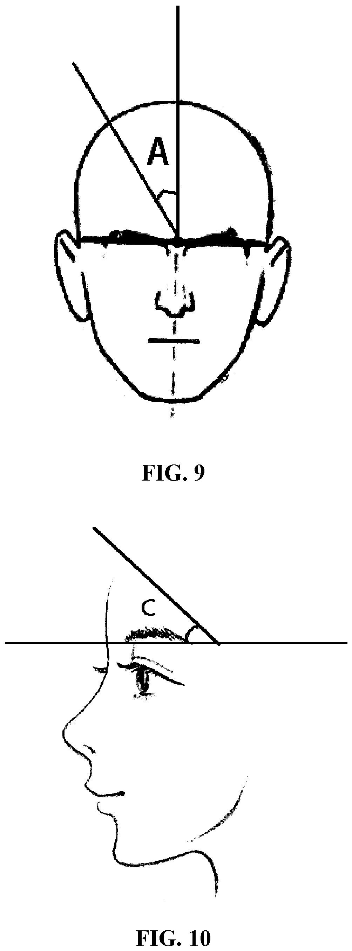

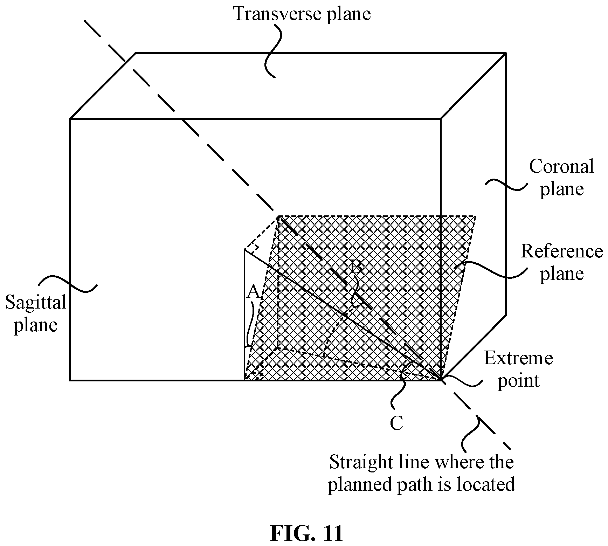

Referring to FIGS. 9 to 11, FIG. 9 is a structure diagram of a viewing angle of a coronal plane of a brain according to an embodiment of the present application, FIG. 10 is a structure diagram of a viewing angle of a sagittal plane of a brain according to an embodiment of the present application, and FIG. 11 illustrates a diagram illustrating a relationship between spatial positions of a second preset angle and a third preset angle according to an embodiment of the present application. As an example, the first preset angle may be, for example, A in FIGS. 9 and 11, the second preset angle may be, for example, B in FIG. 11, and the third preset angle may be, for example, C in FIGS. 10 and 11.

In some other possible implementations, step S203 may include the steps described below.

A reference plane passing through the extreme point is positioned according to the positioning data of the extreme point. An acute included angle formed between the reference plane and a middle sagittal plane of the brain is a first preset angle, and a normal of the reference plane is parallel to any coronal plane of the brain.

An inclined plane passing through the extreme point is positioned. An included angle formed between the inclined plane and any transverse plane of the brain is a third preset angle, a normal of the inclined plane is parallel to any sagittal plane of the brain, and the third preset angle is determined by the first preset angle and the second preset angle.

An intersection line of the reference plane and the inclined plane is determined as the straight line where the path is located.

In this manner, the intersection line of the two planes (the reference plane and the inclined plane) is used for determining the straight line, and the straight line can be quickly positioned from the reference plane.

In some other possible implementations, step S203 may include the steps described below.

A cone with the extreme point as a vertex is positioned according to the positioning data of the extreme point. An axis of the cone is perpendicular to the transverse plane of the brain, and an included angle formed between a bus of the cone and any transverse plane of the brain is a second preset angle.

One of the two intersection lines of the cone and the reference plane is determined as the straight line.

In this manner, using the reference point as the vertex of the (inverted positive) cone forms a space constraint on the axis and the bus of the cone so that the straight line can be quickly located.

Embodiments of the present application further provide a path planning method. The path planning method includes the steps described below.

Positioning data of a nucleus accumbens, a caudate nucleus, a putamen, an anterior limb of an internal capsule and a medial diagonal band of one hemisphere of a brain of a patient is acquired (as long as approximate positions of the nucleus accumbens, the caudate nucleus, the putamen, the anterior limb of the internal capsule and the medial diagonal band can be acquired) according to magnetic resonance image data (MR image data) and CT image data of the brain of the patient.

A midpoint of a connecting line between an anterior commissure and a posterior commissure is set as a reference point to acquire a two-dimensional image of a coronal plane where the reference point is located.

Grayscale and contrast of the two-dimensional image are adjusted to more clearly determine positions of the above one or more function regions.

An extreme point of a ventral contour of the nucleus accumbens is determined from the two-dimensional image.

A middle sagittal plane between left and right brains is acquired, and a plane pointing to the extreme point is positioned, where an acute included angle formed between the plane and the middle sagittal plane is a first preset included angle.

A straight line is acquired from the plane, where an acute included angle formed between the plane and any transverse plane is a second preset included angle. The straight line is used as a straight line where a path for implanting an electrode lead into an intracranial region is located.

Embodiments of the present application further provide a path planning apparatus. Specific implementations of the path planning apparatus are the same as the implementations that are recorded in the above method implementation and the technical effects that are achieved in the above method implementation, and some contents are not repeated.

The path planning apparatus includes a processor configured to perform the operation described below.

A path through which an electrode lead is implanted into an intracranial region of a patient is planned, where the planned path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient.

The electrode lead is configured to deliver electrical stimulation to the anterior limb of the internal capsule and the nucleus accumbens.

In this manner, an implantation path of a particular electrode lead is planned, and the electrode lead is implanted into one hemisphere of the brain of the patient (for example, a left hemisphere or a right hemisphere) according to the path so that joint electrical stimulation can be performed on a nucleus accumbens and an anterior limb of an internal capsule of this hemisphere to treat the patient. The path planned by the path planning apparatus enables the electrode lead to be in contact with an anterior limb of an internal capsule and a nucleus accumbens of one hemisphere. In this manner, the electrode lead can simultaneously deliver electrical stimulation to the anterior limb of the internal capsule and the nucleus accumbens, and a joint electrical stimulation therapy is achieved. In a DBS field, a doctor performed an electrical stimulation therapy on only one particular brain tissue in the past. Therefore, after one electrode lead was implanted into an intracranial region of a patient, even though multiple electrode patches may be on the electrode lead, these electrode patches were used for delivering electrical stimulation to the same brain tissue (nucleus) and cannot simultaneously deliver electrical stimulation to multiple brain tissues, and no joint electrical stimulation therapy was performed on two particular brain tissues (an anterior limb of an internal capsule and a nucleus accumbens). The path planning apparatus provided in the embodiment of the present application can plan the implantation path of the electrode lead so that after the electrode lead is implanted into the intracranial region of the patient, a joint electrical stimulation therapy can be performed on an anterior limb of an internal capsule and a nucleus accumbens of one hemisphere of the brain, having a relatively good therapeutical effect on diseases such as addictive behavior, depressive disorder and obsessive-compulsive disorder.

In some possible implementations, the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side.

A disease of the patient includes one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

In this manner, the electrode lead passes through the anterior limb of the internal capsule and the nucleus accumbens in a particular direction, and the joint electrical stimulation for the anterior limb of the internal capsule and the nucleus accumbens is used for the treatment of a particular type of disease, for example, the treatment of multiple mental disorders, thereby effectively alleviating a condition of the patient or even curing a disease of the patient.

In some possible implementations, the processor is configured to plan the path in the manners described below.

Image data of the brain of the patient is acquired, where the image data includes magnetic resonance image data and/or CT image data.

An extreme point of a ventral contour of the nucleus accumbens is positioned according to the image data to obtain positioning data of the extreme point.

The path through which the electrode lead is implanted into the intracranial region of the patient is planned according to the positioning data of the extreme point so that a straight line where the path is located passes through the extreme point from a superior side to an inferior side, from an anterior side to a posterior side and from a lateral side to a medial side.

In this manner, the image data is used for positioning the extreme point of the ventral contour of the nucleus accumbens, and the extreme point is used for planning the implantation path of the electrode lead so that the straight line where the planned path is located passes through the extreme point.

In some possible implementations, the processor is configured to position the straight line in the manners described below.

A set point of the anterior limb of the internal capsule is positioned according to the image data to obtain positioning data of the set point.

The straight line is positioned according to the positioning data of the set point and the positioning data of the extreme point so that the straight line passes through the set point and the extreme point.

In this manner, the set point of the anterior limb of the internal capsule and the extreme point of the nucleus accumbens are used for forming a constraint on the path, and the implantation path of the electrode lead is planned in a two-point-one-line manner so that the electrode lead sequentially passes through the anterior limb of the internal capsule and the nucleus accumbens in an implantation process from a superior side to an inferior side, from an anterior side to a posterior side and from a lateral side to a medial side.

In some possible implementations, the processor is configured to position a set point of the anterior limb of the internal capsule in the manners described below.

A segmentation result of the anterior limb of the internal capsule is acquired according to the image data, where the segmentation result of the anterior limb of the internal capsule is configured to indicate multiple regions of the anterior limb of the internal capsule.

One of the multiple regions is determined as a target region according to a projection relationship between each of the multiple regions and a frontal lobe so that a white matter tract traveling in the target region can be projected into a target prefrontal cortex, where the target frontal cortex is a frontal cortex associated with the disease of the patient.

The set point of the anterior limb of the internal capsule is positioned from the target region.

In this manner, the image data is used for acquiring the segmentation result of the anterior limb of the internal capsule, and the segmentation result of the anterior limb of the internal capsule is used for positioning the set point of the anterior limb of the internal capsule. When the implantation path passes through the set point, the electrode lead can release electrical stimulation energy to a contact portion (the target region) of the anterior limb of the internal capsule with the electrode lead. This energy is projected into the target prefrontal cortex via the white matter tract in the target region and accurately acts on a particular prefrontal cortex associated with the disease of the patient, thereby achieving the precise treatment of a mental disorder.

In some possible implementations, the processor is configured to position the straight line in the manners described below.

The straight line is enabled to be in a reference plane, and an acute included angle formed between the straight line and any transverse plane of the brain is enabled to be a second preset angle.

The reference plane satisfies the following conditions: the extreme point is in the reference plane, an acute included angle formed between the reference plane and any sagittal plane of the brain is a first preset angle, and a normal of the reference plane is parallel to any coronal plane of the brain.

In this manner, the straight line where the path is located is located in a particular reference plane, and a particular acute included angle (the second preset angle) is formed between the straight line and any transverse plane of the brain. The setting of the reference plane needs to satisfy multiple conditions. Not only the extreme point is set in the reference plane, but also a particular acute included angle (the first preset angle) is formed between the reference plane and any sagittal plane, and the normal of the reference plane is parallel to the coronal plane, that is, a projection of the reference plane on the coronal plane is a straight line. In this manner, the straight line where the planned path is located can pass through the anterior limb of the internal capsule and the nucleus accumbens in a particular direction and at a particular angle. Therefore, the electrode lead implanted according to such a path can pass through the anterior limb of the internal capsule and the nucleus accumbens in the particular direction and at the particular angle, thereby performing the joint electrical stimulation therapy on the two brain tissues.

In some possible implementations, the first preset angle is not less than 5 degrees and is not greater than 85 degrees, and the second preset angle is not less than 15 degrees and is less than 90 degrees.

In this manner, since a brain structure varies with each individual and a brain structure of each person is different, the first preset angle and the second preset angle may be values within a relatively wide value range, provided that joint electrical stimulation can be performed on an anterior limb of an internal capsule and a nucleus accumbens of one hemisphere of the brain of the patient.

In some possible implementations, the first preset angle is not less than 15 degrees and is not greater than 45 degrees, and the second preset angle is not less than 40 degrees and is not greater than 85 degrees.

In this manner, although a brain structure of each person is different, brain structures of most people are relatively similar. In terms of probability, a more accurate angle range can be preferentially used to quickly determine a direction of the implantation path, which is sufficient to cope with a general situation of most people. For a very small number of people whose brain structures are very different from those of ordinary people, a wider range can be enabled as an alternative to help a doctor plan a path for these few people to implant electrode leads.

In some possible implementations, the processor is configured to determine the first preset angle and the second preset angle in the manners described below.

A set point of the anterior limb of the internal capsule is positioned according to the image data to obtain positioning data of the set point.

The first preset angle and the second preset angle are determined according to the positioning data of the set point and the positioning data of the extreme point.

In this manner, before the first preset angle, the second preset angle and the extreme point are used for determining the implantation path, the set point of the anterior limb of the internal capsule and the extreme point of the nucleus accumbens are used as the constraint, the value range of the first preset angle and the second preset angle are significantly narrowed, and a range where the implantation path of the electrode lead is located in the implantation process is significantly narrowed. In particular, when the first preset angle and the second preset angle may be values within a relatively wide value range, an amount of data for calculating all feasible solutions of the first preset angle and the second preset angle can be significantly reduced.

In some possible implementations, the processor is configured to position the extreme point of the ventral contour of the nucleus accumbens in the manners described below.

A reference point is positioned according to the image data, and a two-dimensional image of the brain corresponding to the reference point is generated, where the two-dimensional image of the brain corresponding to the reference point includes one or more of a two-dimensional image of a coronal plane, a two-dimensional image of a sagittal plane and a two-dimensional image of a transverse plane.

The extreme point of the ventral contour of the nucleus accumbens is positioned by using the two-dimensional image of the brain corresponding to the reference point.

In this manner, the image data is used for positioning the reference point. The reference point may be, for example, an anterior commissure, a posterior commissure or a midpoint of the anterior commissure and the posterior commissure. After the reference point is positioned, a coronal plane, a sagittal plane and a transverse plane of the brain corresponding to the reference point can be positioned, that is, a coronal plane passing through the reference point (in other words, the reference point is in the coronal plane), a sagittal plane passing through the reference point and a transverse plane passing through the reference point, and three-dimensional image data is used for generating one or more of the two-dimensional images corresponding to the coronal plane, the sagittal plane and the transverse plane. Since the ventral contour of the nucleus accumbens presents a parabola-like shape in the above two-dimensional image (an opening of the parabola is upward and outward), the extreme point of the ventral contour of the nucleus accumbens can be positioned from the parabola. Moreover, the step of positioning the extreme point may be achieved in an intelligent recognition or manual calibration manner. For doctors, the manner for positioning the extreme point is very intuitive, and the extreme point can be quickly calibrated according to experience. In the intelligent recognition manner, the contour of the nucleus accumbens can be quickly recognized through an edge detection algorithm and/or an image segmentation algorithm, thereby positioning the extreme point of the ventral contour of the nucleus accumbens. The path planning apparatus provided in the embodiment of the present application generates the two-dimensional image by using the reference point and positions the extreme point by using the two-dimensional image. This manner of positioning the extreme point has a simple calculation process, a small calculation amount and a fast calculation speed and consumes less calculation resources.

In some possible implementations, the reference point is a midpoint of a connecting line between an anterior commissure and a posterior commissure of the brain.

Alternatively, the reference point is an anterior commissure of the brain.

Alternatively, the reference point is a posterior commissure of the brain.

In this manner, using the anterior commissure of the brain, the posterior commissure of the brain or the midpoint of the connecting line between the anterior commissure and the posterior commissure of the brain as the reference point has the advantages of simple operation and easy implementation in the intelligent recognition manner or the manual calibration manner. For doctors, the manner for positioning the reference point is very intuitive, and the reference point can be quickly calibrated according to experience. In the intelligent recognition manner, a contour of an entire brain can be quickly obtained through the edge detection algorithm, and the anterior commissure and the posterior commissure are positioned from the contour of the entire brain, thereby positioning the reference point.

In some possible implementations, the processor is configured to position the extreme point of the ventral contour of the nucleus accumbens in the manners described below.

An adjustment operation for each two-dimensional image is received, and in response to the adjustment operation, one or more of the following parameters of the each two-dimensional image are adjusted: brightness, contrast, saturation, exposure, hue, grayscale and scaling.

The extreme point of the ventral contour of the nucleus accumbens is positioned by using the adjusted two-dimensional image.

In this manner, doctors can manually adjust the parameter of the two-dimensional image. Adjusting the parameter can change a display effect of the two-dimensional image, thereby facilitating determining the extreme point from the two-dimensional image. The adjusted parameter is not limited in the embodiment of the present application and may be, for example, one or more of multiple parameters that affect the display effect. Apparently, the adjustment to one or more of these parameters can make the ventral contour of the nucleus accumbens more convex and clearer (in other words, the contrast is higher) so that the positioned extreme point is more accurate and reliable, the positioning speed is faster and the positioning efficiency is high, thereby improving overall path planning efficiency.

In some possible implementations, the adjustment operation is an operation of adjusting a parametric curve and is configured to adjust the brightness, the contrast or the grayscale of the each two-dimensional image.

Alternatively, the adjustment operation is an operation of adjusting a parameter progress bar and is configured to adjust the brightness, the contrast, the saturation, the exposure, the hue, the grayscale and the scaling of the each two-dimensional image.

Alternatively, the adjustment operation is an operation of adjusting a parameter value and is configured to adjust the brightness, the contrast, the saturation, the exposure, the hue, the grayscale and the scaling of the each two-dimensional image.

In this manner, doctors are allowed to adjust the brightness or the contrast in a manner of adjusting the parametric curve, the doctors are allowed to adjust multiple parameters in a manner of adjusting the parameter progress bar, or the doctors are allowed to adjust the multiple parameters in a manner of adjusting the parameter value so that the doctors can select a manner of adjusting the parameters according to preferences of the doctors to quickly adjust the display effect of the two-dimensional image, thereby further improving the path planning efficiency.

In some possible implementations, the processor is configured to position the extreme point of the ventral contour of the nucleus accumbens in the manners described below.

A three-dimensional model of the brain of the patient is generated according to the image data.

The extreme point of the ventral contour of the nucleus accumbens is positioned by using the three-dimensional model.

In this manner, the image data is used for generating the three-dimensional model of the brain, and the three-dimensional model is used for positioning the extremum point. The manner of positioning the extreme point may be intelligent recognition or manual calibration. When the manual calibration is used, compared with the image data and the two-dimensional image, the three-dimensional model has higher visibility, and doctors can more conveniently and quickly calibrate the extreme point. When the intelligent recognition is used, compared with the image data and the two-dimensional image (data completion needs to be performed on the image data or the two-dimensional image in manners such as an interpolation algorithm to obtain the extreme point), the three-dimensional model has a continuous shape (in other words, a contour), and the extreme point can be quickly calculated without the data completion. Moreover, the calculation amount is small, the calculation speed is fast, the calculation efficiency is high, and less calculation resources are consumed.

In some possible implementations, the processor is configured to plan the path in the manners described below.

A three-dimensional model of the brain of the patient is generated according to the image data.

The three-dimensional model is displayed by using a display device, where the three-dimensional model includes a three-dimensional structure of the nucleus accumbens and a three-dimensional structure of the anterior limb of the internal capsule.

A path planning operation is received by using an interaction device, and in response to the path planning operation, the path is planned.

In this manner, in the three-dimensional model, both the nucleus accumbens and the anterior limb of the internal capsule can be displayed. Doctors design a surgical path according to three-dimensional structures of two displayed target sites (the nucleus accumbens and the anterior limb of the internal capsule).

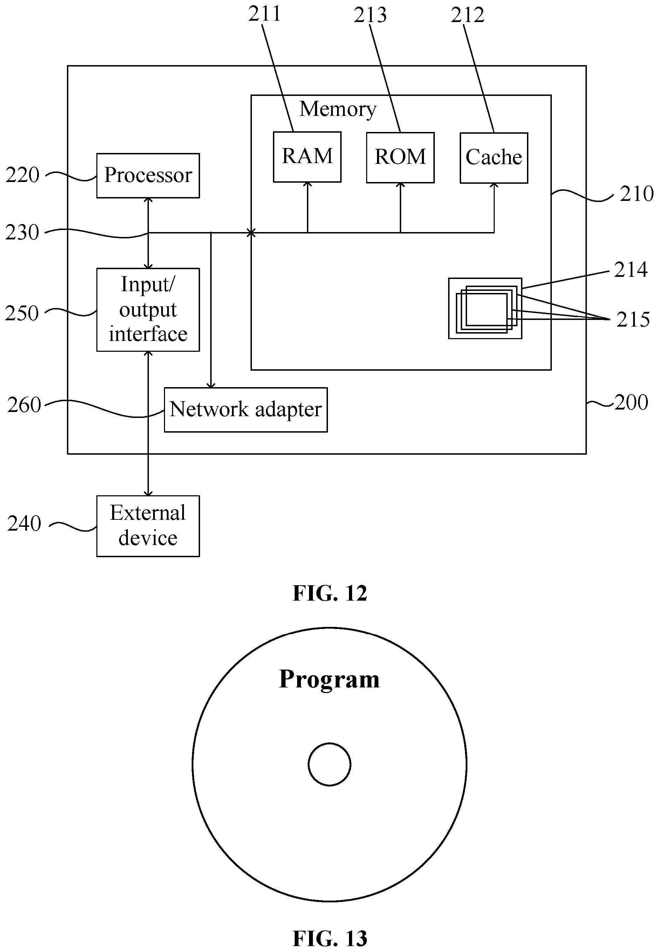

Referring to FIG. 12, FIG. 12 is a block diagram of a path planning apparatus 200 according to an embodiment of the present application. The path planning apparatus 200 may include, for example, at least one memory 210, at least one processor 220 and a bus 230 connected to different platform systems.

The memory 210 may include a readable medium formed by a volatile memory, such as a random-access memory (RAM) 211 and/or a cache 212, and may further include a read-only memory (ROM) 213.

The memory 210 further stores a computer program. The computer program may be executed by the processor 220 so that the processor 220 implements functions of any one of the above path planning apparatuses or steps of any one of the above path planning methods. Specific implementations of the functions and the steps are the same as the implementations that are recorded in the above method implementation and the technical effects that are achieved in the above method implementation, and some contents are not repeated.

The memory 210 may further include a utility 214 having at least one program module 215. Such program modules 215 include, but are not limited to, an operating system, one or more application programs, and other program modules and program data. Each or a certain combination of these examples may include implementation of a network environment.

Correspondingly, the processor 220 may execute the above computer program and may execute the utility 214.

The processor 220 may use one or more application-specific integrated circuits (ASICs), DSPs, programmable logic devices (PLDs), complex programmable logic devices (CPLDs), field-programmable gate arrays (FPGAs) or other electronic elements.

The bus 230 may represent one or more of several types of bus architectures, including a memory bus or memory path planning apparatus, a peripheral bus, an Accelerated Graphics Port and a processor, or represents a local bus using any one of multiple bus architectures.

The path planning apparatus 200 may further communicate with one or more external devices 240 (e.g., a keyboard, a pointing device, or a Bluetooth device). The path planning apparatus 200 may further communicate with one or more devices that can interact with the path planning apparatus 200, and/or with any device (e.g., a router card or a modem) that enables the path planning apparatus 200 to communicate with one or more other computing devices. These communications may be performed through an input/output interface 250. Moreover, the path planning apparatus 200 may communicate with one or more networks (such as a local area network (LAN), a wide area network (WAN), and/or a public network, for example, the Internet) through a network adapter 260. The network adapter 260 may communicate with other modules of the path planning apparatus 200 by using the bus 230. It is to be understood that although not shown in the figure, other hardware and/or software modules may be used in conjunction with the path planning apparatus 200. The other hardware and/or software modules include, but are not limited to, microcode, a terminal driver, a redundant processor, an external disk drive array, an RAID system, a tape driver, a data backup storage platform and the like.

Embodiments of the present application further provide a computer-readable storage medium storing a computer program, where the computer program, when executed by a processor, implements functions of any one of the above path planning apparatuses or implements steps of any one of the above path planning methods. Specific implementations of the functions and the steps are the same as the implementations that are recorded in the above path planning method and the technical effects that are achieved in the above path planning method, and some contents are not repeated.

Referring to FIG. 13, FIG. 13 is a structure diagram of a program product for implementing a path planning method according to an embodiment of the present application. The program product may use a portable compact disk read-only memory (CD-ROM), may include a program code and may run on terminal devices such as a personal computer. However, the program product of the present invention is not limited thereto. In the embodiment of the present application, the readable storage medium may be any tangible medium including or storing a program. The program may be used by or used in conjunction with an instruction execution system, apparatus, or element. The program product may use one readable medium or any combination of multiple readable media. The readable medium may be a readable signal medium or a readable storage medium. The readable storage medium may be, but is not limited to, an electrical, magnetic, optical, electromagnetic, infrared or semiconductor system, apparatus or device, or any combination thereof. More specific examples of each readable storage medium include (non-exhaustive list): an electrical connection having one or more leads, a portable disk, a hard disk, a random access memory (RAM), a read-only memory (ROM), an erasable programmable read-only memory (EPROM or flash memory), an optical fiber, a portable compact disk read-only memory (CD-ROM), an optical memory device, a magnetic memory device, or any suitable combination thereof.

The computer-readable storage medium may include a data signal propagated in a baseband or as part of a carrier. Readable program codes are carried in the data signal. The data signal propagated in this manner may be in multiple forms and includes, but is not limited to, an electromagnetic signal, an optical signal, or any suitable combination thereof. The readable storage medium may also be any readable medium, and the readable medium may send, propagate or transmit a program used by or in combination with an instruction execution system, apparatus or device. Program code contained in the readable storage medium may be transmitted via any suitable medium. The medium includes, but is not limited to, the leadless, a lead, an optical cable, radio frequency (RF), or the like, or any suitable combination thereof. Program codes for performing the operations of the present invention may be written in one programming language or any combination of multiple programming languages, the programming languages including object-oriented programming languages such as Java, C++ and further including conventional procedural programming languages such as C programming language or similar programming languages. Program codes may be executed entirely on a user computing device, executed partly on a user computing device, executed as a stand-alone software package, executed partly on a user computing device and partly on a remote computing device or executed entirely on a remote computing device or a server. In the case where the remote computing device is involved, the remote computing device may be connected to the user computing device via any type of network including a local area network (LAN) or a wide area network (WAN) or may be connected to an external computing device (for example, via the Internet provided by an Internet service provider).

In terms of object, performance, progress and novelty, the present application is described. The above specification and specification drawings are merely better embodiments of the present application and are not intended to limit the present application. Therefore, all structures, apparatuses and features that are similar or identical to those of the present application, that is, any equivalent replacement or modification made according to the patent application scope of the present application should fall within the patent application protection scope of the present application.

## Claims

1. A path planning apparatus, comprising a processor configured to:
plan a path through which an electrode lead is implanted into an intracranial region of a patient, wherein the path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient; and
the electrode lead is configured to deliver electrical stimulation to the anterior limb of the internal capsule and the nucleus accumbens.

2. The apparatus according to claim 1, wherein the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side; and
a disease of the patient comprises one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

3. The apparatus according to claim 2, wherein the processor is configured to plan the path in the following manners:
acquiring image data of the brain of the patient, wherein the image data comprises at least one of magnetic resonance image data or CT image data;
positioning, according to the image data, an extreme point of a ventral contour of the nucleus accumbens to obtain positioning data of the extreme point; and
planning, according to the positioning data of the extreme point, the path through which the electrode lead is implanted into the intracranial region of the patient so that a straight line where the path is located passes through the extreme point from a superior side to an inferior side, from an anterior side to a posterior side and from a lateral side to a medial side.

4. The apparatus according to claim 3, wherein the processor is configured to position the straight line in the following manners:
positioning, according to the image data, a set point of the anterior limb of the internal capsule to obtain positioning data of the set point; and
positioning, according to the positioning data of the set point and the positioning data of the extreme point, the straight line so that the straight line passes through the set point and the extreme point.

5. The apparatus according to claim 4, wherein the processor is configured to position a set point of the anterior limb of the internal capsule in the following manners:
acquiring, according to the image data, a segmentation result of the anterior limb of the internal capsule, wherein the segmentation result of the anterior limb of the internal capsule is configured to indicate a plurality of regions of the anterior limb of the internal capsule;
determining, based on a projection relationship between each of the plurality of regions and a frontal lobe, one of the plurality of regions as a target region so that a white matter tract traveling in the target region can be projected into a target prefrontal cortex, wherein the target frontal cortex is a frontal cortex associated with the disease of the patient; and
positioning the set point of the anterior limb of the internal capsule from the target region.

6. The apparatus according to claim 3, wherein the processor is configured to position the straight line in the following manners:
enabling the straight line to be in a reference plane, and enabling an acute included angle formed between the straight line and any transverse plane of the brain to be a second preset angle;
wherein the reference plane satisfies the following conditions: the extreme point is in the reference plane, an acute included angle formed between the reference plane and any sagittal plane of the brain is a first preset angle, and a normal of the reference plane is parallel to any coronal plane of the brain.

7. The apparatus according to claim 6, wherein the first preset angle is not less than 5 degrees and is not greater than 85 degrees, and the second preset angle is not less than 15 degrees and is less than 90 degrees.

8. The apparatus according to claim 7, wherein the first preset angle is not less than 15 degrees and is not greater than 45 degrees, and the second preset angle is not less than 40 degrees and is not greater than 85 degrees.

9. The apparatus according to claim 8, wherein the processor is configured to determine the first preset angle and the second preset angle in the following manners:
positioning, according to the image data, a set point of the anterior limb of the internal capsule to obtain positioning data of the set point; and
determining, according to the positioning data of the set point and the positioning data of the extreme point, the first preset angle and the second preset angle.

10. The apparatus according to claim 3, wherein the processor is configured to position the extreme point of the ventral contour of the nucleus accumbens in the following manners:
positioning, according to the image data, a reference point, and generating a two-dimensional image of the brain corresponding to the reference point, wherein the two-dimensional image of the brain corresponding to the reference point comprises one or more of a two-dimensional image of a coronal plane, a two-dimensional image of a sagittal plane and a two-dimensional image of a transverse plane; and
positioning, by using the two-dimensional image of the brain corresponding to the reference point, the extreme point of the ventral contour of the nucleus accumbens.

11. The apparatus according to claim 10, wherein the reference point is a midpoint of a connecting line between an anterior commissure and a posterior commissure of the brain; or
the reference point is an anterior commissure of the brain; or
the reference point is a posterior commissure of the brain.

12. The apparatus according to claim 10, wherein the processor is configured to position the extreme point of the ventral contour of the nucleus accumbens in the following manners:
receiving an adjustment operation for the two-dimensional image, and adjusting, in response to the adjustment operation, one or more of the following parameters of the each two-dimensional image: brightness, contrast, saturation, exposure, hue, grayscale and scaling; and
positioning, by using the adjusted two-dimensional image, the extreme point of the ventral contour of the nucleus accumbens.

13. The apparatus according to claim 12, wherein the adjustment operation is an operation of adjusting a parametric curve and is configured to adjust the brightness, the contrast or the grayscale of the two-dimensional image; or
the adjustment operation is an operation of adjusting a parameter progress bar and is configured to adjust the brightness, the contrast, the saturation, the exposure, the hue, the grayscale and the scaling of the two-dimensional image; or
the adjustment operation is an operation of adjusting a parameter value and is configured to adjust the brightness, the contrast, the saturation, the exposure, the hue, the grayscale and the scaling of the two-dimensional image.

14. The apparatus according to claim 3, wherein the processor is configured to position the extreme point of the ventral contour of the nucleus accumbens in the following manners:
generating, according to the image data, a three-dimensional model of the brain of the patient; and
positioning, by using the three-dimensional model, the extreme point of the ventral contour of the nucleus accumbens.

15. The apparatus according to claim 2, wherein the processor is configured to plan the path in the following manners:
generating, according to image data, a three-dimensional model of the brain of the patient;
displaying, by using a display device, the three-dimensional model, wherein the three-dimensional model comprises a three-dimensional structure of the nucleus accumbens and a three-dimensional structure of the anterior limb of the internal capsule; and
receiving, by using an interaction device, a path planning operation, and planning, in response to the path planning operation, the path.

16. A path planning method, comprising:
planning a path through which an electrode lead is implanted into an intracranial region of a patient, wherein the path satisfies the following condition: if the electrode lead is implanted into the intracranial region of the patient according to the path, the electrode lead is capable of being in contact with both an anterior limb of an internal capsule and a nucleus accumbens of one hemisphere of a brain of the patient; and
the electrode lead is configured to deliver electrical stimulation to the anterior limb of the internal capsule and the nucleus accumbens.

17. The method according to claim 16, wherein the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side; and
a disease of the patient comprises one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

18. The method according to claim 17, wherein planning the path through which the electrode lead is implanted into the intracranial region of the patient comprises:
acquiring image data of the brain of the patient, wherein the image data comprises at least one of magnetic resonance image data or CT image data;
positioning, based on the image data, an extreme point of a ventral contour of the nucleus accumbens to obtain positioning data of the extreme point; and
planning, based on the positioning data of the extreme point, the path through which the electrode lead is implanted into the intracranial region of the patient so that a straight line where the path is located passes through the extreme point from a superior side to an inferior side, from an anterior side to a posterior side and from a lateral side to a medial side.

19. The method according to claim 18, wherein a process of positioning the straight line comprises:
positioning, based on the image data, a set point of the anterior limb of the internal capsule to obtain positioning data of the set point; and
positioning, based on the positioning data of the set point and the positioning data of the extreme point, the straight line so that the straight line passes through the set point and the extreme point.

20. The method according to claim 19, wherein positioning, based on the image data, the set point of the anterior limb of the internal capsule comprises:
acquiring, based on the image data, a segmentation result of the anterior limb of the internal capsule, wherein the segmentation result of the anterior limb of the internal capsule is configured to indicate a plurality of regions of the anterior limb of the internal capsule;
determining, based on a projection relationship between each of the plurality of regions and a frontal lobe, one of the plurality of regions as a target region so that a white matter tract traveling in the target region can be projected into a target prefrontal cortex, wherein the target frontal cortex is a frontal cortex associated with the disease of the patient; and
positioning the set point of the anterior limb of the internal capsule from the target region.

21. The method according to claim 18, wherein a process of positioning the straight line comprises:
enabling the straight line to be in a reference plane, and enabling an acute included angle formed between the straight line and any transverse plane of the brain to be a second preset angle;
wherein the reference plane satisfies the following conditions: the extreme point is in the reference plane, an acute included angle formed between the reference plane and any sagittal plane of the brain is a first preset angle, and a normal of the reference plane is parallel to any coronal plane of the brain.

22. The method according to claim 21, wherein the first preset angle is not less than 5 degrees and is not greater than 85 degrees, and the second preset angle is not less than 15 degrees and is less than 90 degrees.

23. The method according to claim 22, wherein the first preset angle is not less than 15 degrees and is not greater than 45 degrees, and the second preset angle is not less than 40 degrees and is not greater than 85 degrees.

24. The method according to claim 23, wherein a process of determining the first preset angle and the second preset angle comprises:
positioning, based on the image data, a set point of the anterior limb of the internal capsule to obtain positioning data of the set point; and
determining, based on the positioning data of the set point and the positioning data of the extreme point, the first preset angle and the second preset angle.

25. The method according to claim 18, wherein positioning, based on the image data, the extreme point of the ventral contour of the nucleus accumbens comprises:
positioning, based on the image data, a reference point, and generating a two-dimensional image of the brain corresponding to the reference point, wherein the two-dimensional image of the brain corresponding to the reference point comprises one or more of a two-dimensional image of a coronal plane, a two-dimensional image of a sagittal plane and a two-dimensional image of a transverse plane; and
positioning, by using the two-dimensional image of the brain corresponding to the reference point, the extreme point of the ventral contour of the nucleus accumbens.

26. The method according to claim 25, wherein the reference point is a midpoint of a connecting line between an anterior commissure and a posterior commissure of the brain; or
the reference point is an anterior commissure of the brain; or
the reference point is a posterior commissure of the brain.

27. The method according to claim 25, wherein positioning, by using the two-dimensional image of the brain corresponding to the reference point, the extreme point of the ventral contour of the nucleus accumbens comprises:
receiving an adjustment operation for the two-dimensional image, and adjusting, in response to the adjustment operation, one or more of the following parameters of the two-dimensional image: brightness, contrast, saturation, exposure, hue, grayscale and scaling; and
positioning, by using the adjusted two-dimensional image, the extreme point of the ventral contour of the nucleus accumbens.

28. The method according to claim 27, wherein the adjustment operation is an operation of adjusting a parametric curve and is configured to adjust the brightness, the contrast or the grayscale of the two-dimensional image; or
the adjustment operation is an operation of adjusting a parameter progress bar and is configured to adjust the brightness, the contrast, the saturation, the exposure, the hue, the grayscale and the scaling of the two-dimensional image; or
the adjustment operation is an operation of adjusting a parameter value and is configured to adjust the brightness, the contrast, the saturation, the exposure, the hue, the grayscale and the scaling of the two-dimensional image.

29. The method according to claim 18, wherein positioning, based on the image data, the extreme point of the ventral contour of the nucleus accumbens comprises:
generating, based on the image data, a three-dimensional model of the brain of the patient; and
positioning, by using the three-dimensional model, the extreme point of the ventral contour of the nucleus accumbens.

30. The method according to claim 17, wherein planning the path through which the electrode lead is implanted into the intracranial region of the patient comprises:
generating, based on image data, a three-dimensional model of the brain of the patient;
displaying, by using a display device, the three-dimensional model, wherein the three-dimensional model comprises a three-dimensional structure of the nucleus accumbens and a three-dimensional structure of the anterior limb of the internal capsule; and
receiving, by using an interaction device, a path planning operation, and planning, in response to the path planning operation, the path.

31. A surgical system, comprising:
the path planning apparatus according to any one of claims 1 to 15 configured to plan a path through which an electrode lead is implanted into an intracranial region of a patient; and
a surgical apparatus configured to perform a surgery of implanting the electrode lead into the intracranial region of the patient according to the path so that the electrode lead is in contact with an anterior limb of an internal capsule and a nucleus accumbens of one hemisphere of a brain of the patient;
wherein the electrode lead is configured to deliver electrical stimulation to the anterior limb of the internal capsule and the nucleus accumbens.

32. The surgical system according to claim 31, wherein the electrode lead being in contact with the anterior limb of the internal capsule and the nucleus accumbens means that the electrode lead passes through the anterior limb of the internal capsule and the nucleus accumbens from a superior side to an inferior side, from an anterior side to a posterior side and from a lateral side to a medial side; and
a disease of the patient comprises one or more of the following mental disorders: depressive disorder, bipolar disorder, anxiety disorder, post-traumatic stress disorder, obsessive-compulsive disorder, behavior disorder, mood disorder, memory disorder, mental state disorder, drug addiction and autism.

33. A computer-readable storage medium storing a computer program, wherein the computer program, when executed by a processor, implements functions of the path planning apparatus according to any one of claims 1 to 15 or implements steps of the path planning method according to any one of claims 16 to 30.
